(19) Europäisches Patentamt
European Patent Office
Office européen des brevets

(11) Publication number: 0 322 033 A2

(12) # EUROPEAN PATENT APPLICATION

(21) Application number: 88202879.8

(22) Date of filing: 14.12.88

(51) Int. Cl.[4]: C07D 307/12 , C07D 307/14 , C07D 307/18 , C07D 307/42 , C07D 405/04 , C07D 405/14 , C07D 407/12 , C07D 405/12 , C07D 417/04 , A61K 31/34

Claims for the following Contracting States: ES + GR.

(30) Priority: 21.12.87 US 135862

(43) Date of publication of application: 28.06.89 Bulletin 89/26

(84) Designated Contracting States: AT BE CH DE ES FR GB GR IT LI LU NL SE

(71) Applicant: MERCK & CO. INC.
126, East Lincoln Avenue P.O. Box 2000
Rahway New Jersey 07065-0900(US)

(72) Inventor: Biftu, Tesfaye
25 Victorian Drive
Old Bridge New Jersey 07885(US)
Inventor: Hwang, San-Boa
67 Canterbury Drive
Scotch Plains New Jersey 07076(US)
Inventor: Doebber, Thomas W.
2281 Elizabeth Avenue
Scotch Plains New Jersey 07076(US)
Inventor: Beattie, Thomas R.
4 Heather Lane
Scotch Plains New Jersey 07076(US)
Inventor: Shen, Tsung-Ying
303 Ednam Drive
Charlottesville Virginia 22901(US)

(74) Representative: Hesketh, Alan, Dr.
European Patent Department Merck & Co., Inc. Terlings Park Eastwick Road
Harlow Essex, CM20 2QR(GB)

(54) **New 2,5-diaryl tetrahydrofurans and analogs thereof as paf antagonists.**

(57) A class of 2,5-diaryl tetrahydrofuran derivatives are PAF antagonists and are therefore useful in the treatment of various diseases including prevention of platelet aggregation, hypotension, inflammation, asthma, lung edema, adult respiratory distress syndrome, various shock syndromes, cardiovascular disorders and other related skeletal-muscular disorders.

EP 0 322 033 A2

# NEW 2,5-DIARYL TETRAHYDROFURANS AND ANALOGS THEREOF AS PAF ANTAGONISTS

## BACKGROUND OF THE INVENTION

Platelet-activating factor (PAF) has recently been identified as an acetyl glyceryl ether phosphorylcholine (AGEPC), i.e., 1-O-hexadecyl/octadecyl-2-acetyl-sn-glyceryl-3-phosphorylcholine (Hanahan D.J., *et al.*, *J. Biol. Chem.* 255:5514, (1980). Even before its chemical identification, PAF had been linked to various biological activities and pathways making it one of the important mediators responsible for a variety of physiological processes including activation or coagulation of platelets, pathogenesis of immune complex deposition, smooth muscle contraction, inflammation, hypotension, shock, pain, edema as well as respiratory, cardiovascular and intravascular alterations. Since these physiological processes are in turn associated with a large group of diseases, for example, inflammatory disease, cardiovascular disorder, hypotension, shock, psoriasis, allergic and skin diseases, asthma, lung edema, peptic or stomach ulcer, dental pain, and adult respiratory distress syndrome, more and more scientific investigation has been focused on the search of a PAF antagonist or inhibitor for treating or preventing these common diseases.

The compounds of the present invention are specific PAF antagonists. They are similar to a subclass of compounds called lignans which characteristically contain two phenylpropyl groups bonded at the β-carbon. Tetrahydrofuran (THF) derivatives can exist in eight different stereoisomers as shown in Scheme I.

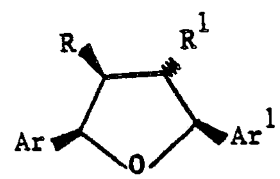
(1)

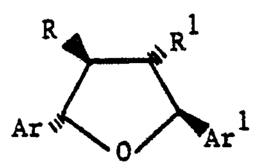
(2)

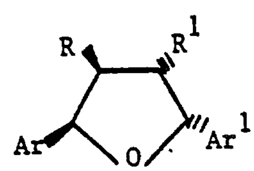
(3)

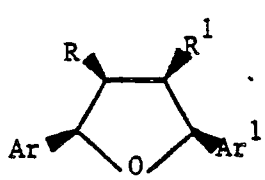
(4)

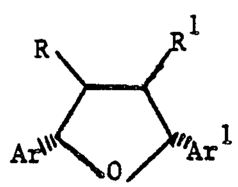
(5)

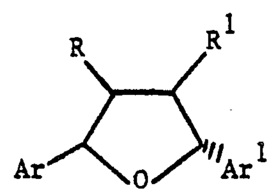
(6)

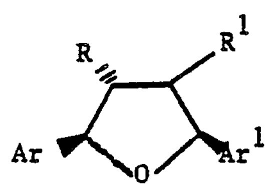
(7)

**Scheme 1**

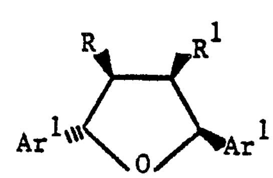
(8)

We have been able to prepare all the possible isomers of the tetrahydrofuran lignan analogs with different substituents and found that activity is stereospecific.

Accordingly, it is the object of the present invention to prepare the most potent isomers of known or novel tetrahydrofuran derivatives as PAF antagonists and use them for the treatment of various diseases including prevention of platelet aggregation, hypotension, inflammation, asthma, lung edema, adult respiratory distress syndrome, various shock syndromes, cardiovascular disorders and other related skeletal-muscular disorders.

Another object of the present invention is to develop processes for the preparation of each and every stereoisomer of the 2,5-diaryltetrahydrofuran analogs.

A further object of the present invention is to provide acceptable pharmaceutical compositions containing one or more of the tetrahydrofuran derivatives and/or analogs as the active ingredient. As PAF antagonists, these novel compositions should be effective in the treatment of various skeletal-muscular

related diseases.

Finally, it is the ultimate object of this invention to provide a method of treatment comprising the administration of a therapeutically sufficient amount of these PAF antagonists to a patient suffering from various skeletal-muscular disorders including inflammation, e.g., osteoarthritis, rheumatoid arthritis and gout, hypotension, shock, psoriasis, allergic or skin diseases, asthma, pain especially dental pain, peptic or stomach ulcer, lung edema, adult respiratory distress syndrome or cardiovascular disorders.

## DETAILED DESCRIPTION OF THE INVENTION

### A. Scope of the Invention

This invention relates to PAF antagonists of the structural formula

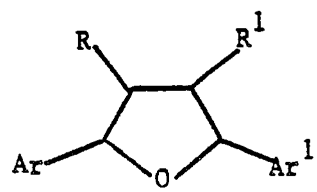

or an optically active enantiomer thereof wherein R and $R^1$ independently are

(a) hydrogen;

(b) haloloweralkyl especially $C_{1-6}$ haloalkyl, for example, trifluoromethyl;

(c) halo especially fluoro;

(d) $CONR^2R^3$ wherein $R^2$ and $R^3$ independently represent

1) H;

2) straight or branched chain $C_{1-20}$ alkyl, e.g., methyl, ethyl, isopropyl, n-butyl, $-CH((CH_2)_4CH_3)_2$, 2-methylpropyl, isobutyl, t-butyl, pentyl or hexyl;

3) $C_{3-8}$ cycloalkyl especially cyclopropyl, cyclopentyl or cyclohexyl;

4) $C_{2-20}$ alkenyl especially $C_{2-6}$ alkenyl such as allyl and $-(CH_2)_4CH=CH_2$, $-(CH_2)_2CH=CH_2$;

5) $C_{2-20}$ alkynyl especially $C_{2-6}$ alkynyl such as $-CH-C\equiv CH$ and $-CH_2CH_2\equiv CH$;

6) aryl of 6 to 14 carbons especially phenyl (Ph) or substituted phenyl as defined below; for example, p-fluorophenyl;

7) aralkyl especially $C_{1-6}$ alkylphenyl and $C_{1-6}$ alkyl-substituted phenyl such as benzyl, $-(CH_2)_3Ph$, $-(CH_2)_6Ph$, and $-CH_2$-p-fluorophenyl;

(e) loweralkenyl especially $C_{1-6}$ alkenyl e.g., vinyl, allyl, $CH_3CH=CH-CH_2-CH_2$, or $CH_3(CH_2)_3CH=CH-$;

(f) $-COR^2$;

(g) $-CH_2OR^2$;

(h) loweralkynyl especially $C_{1-6}$ alkynyl e.g., $-C\equiv CH$;

(i) $-CH_2NR^2R^3$;

(j) $-CH_2SR^2$;

(k) $=O$; or

(l) $-OR^2$;

(m) $-R^2$;

Ar and $Ar^1$ are the same or different from each other and are:

(a) phenyl or substituted phenyl or formula

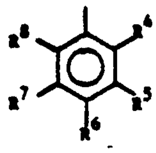

where $R^4$-$R^8$ independently represent:

1) $R^2$;

2) YO-; wherein Y is $-R^2$ or $Y^1$ where $Y^1$ represents

(1) $-(CH_2)_{1-6}\ O(CO)OR^2$;

(2)

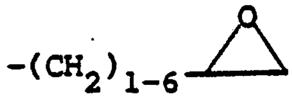 ;

(3) $-(CH_2)_{1-6}$-$C_{3-8}$ cycloalkyl such as $-CH_2$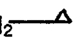, $(CH_2)_3$-cyclohexyl, and $(CH_2)_3$-cyclopentyl;

(4) $-(CH_2)_{1-6}$-$COOR^2$;

(5) $-(CH_2)_{1-6}OR^2$ especially $-(CH_2)_3OH$, $-CH_2CH(OH)CH_2OH$, $-(CH_2)_4CH(OH)CH_3$, $-CH_2CH_2OCH_2CH_3$; $-CH_2CH(OH)CH_3$, $-(CH_2)_6OH$, $CH_2CH(OCH_3)CH_3$, $-CH_2OH$, or $-CH(OCH_3)Ph$ wherein Ph represents phenyl or substituted phenyl;

(6) $-(CH_2)_{1-6}Ph$;

(7)

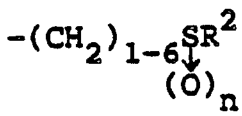

wherein n is 0, 1 or 2, for example, $-CH_2CH_2SO_2C_6H_5$;

(8) haloloweralkyl especially halo-$C_{1-6}$ alkyl such as $-CF_3$, $-CH_2CH(F)CH_3$, $-CF_2CF_3$, $-(CH_2)_5CH_2F$, $-(CH_2)_3Cl$, $-CH_2CH_2Br$ or $(CH_2)_4Br$;

(9) $-(CH_2)_{1-7}OSO_2R^2$ such as $(CH_2)_7OSO_2CH_3$; or

(10) $-(CH_2)_{1-6}NR^2R^3$;

3)

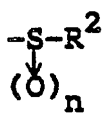

for example, $-CH_2$-S-$CH_3$ and $-CH_2SO_2CH_3$;

4) $-S(O)_n$-$(CH_2)_{1-6}OR^2$ especially $-SO_2CH_2CH_2CH_2OH$, $-SO_2CH_2CH_2OH$;

5) $-OCOCF_3$;

6)

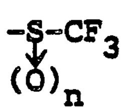 ;

7) $-OCOR_2$ such as

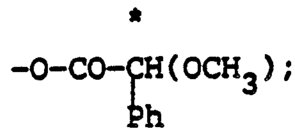

8) $Y^1$;
9) $R^2R^3N$-;
10)

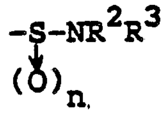

such as $-SO_2NH_2$ $-SO_2N(CH_3)_2$;
11) $COOR^2$;
12) $-CONR^2R^3$;
13) $-NR^2COR^3$;
14) $-OCONR^2R^3$;
15) $-CR^2R^3R^9$ wherein $R^9$ is $R^2$ and can be the same as or different from $R^2$;
16) $-(CH_2)_{1-6}OCOR^2$ especially $-CH_2OCOR^2$ such as $-CH_2OCOCH_3$;
17) $-(CH_2)_{1-6}N(R^2)COR^3$ such as $-CH_2N(CH_3)COCH_3$ and $-CH_2NHCOCH_3$;
18) $-NHCH_2COOR^2$;
19) $-CONR^2COR^3$;
20) $-(CH_2)_{1-6}NR^2R^3$ such as $-CH_2NH_2$;
21) halo such as -F, -Cl, -Br and -I;
22) $-\overset{+}{N}R^2R^3R^9X^-$ wherein $X^-$ is an anion, for examples, $-Cl^-$, $-Br^-$, $I^-$ and $OH^-$;
23) $-(CH_2)_{1-6}\overset{+}{N}R^2R^3R^9X^-$;
24) $-NR^2SO_2R^3$ such as $-NHSO_2CH_3$;
25) $-COR^2$;
26) $-NO_2$;
27) -CN;
28) $-(CH_2)_{1-6}CN$ such as $-CH_2CN$ and $-(CH_2)_6CN$;
29) $-N_3$;
30) $-(CH_2)_{1-6}N_3$;
31) $-CONR^2(CH_2)_{1-6}NR^3R^9$ especially $-CONHCH_2CH_2N(CH_3)_2$;
32) $-PO_3R^2R^3$;
33) $-(CH_2)_{1-6}PO_3R^2R^3$;
34) $-PO_3(R^2)(CH_2)_{1-6}NR^3R^9$;
35) $-R^4$-$R^5$, $R^5$-$R^6$, $R^6$-$R^7$ and $R^7$-$R^8$ joined together and form a bridge, for example, $-OCH_2O-$, $-OCH_2CH_2O-$, $-OCH_2CH_2N=$ or $-SO_2CH_2CH_2O-$;
36) $S(O)_n(CH_2)_{1-6}$-CO-$OR^2$ such as $SO_2CH_2CH_2COOCH_3$;
37)

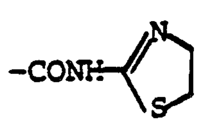 ;

38)

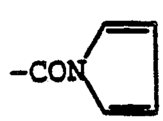 ;

39) $-(CH_2)_{1-6}$ pyridyl especially $-(CH_2)_3$-(3-pyridyl) and $-CH_2$-(2-pyridyl);
40) $-SO_2NH(CH_2)_{1-6}$-(2-pyridyl);
41)

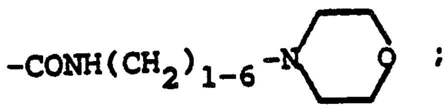

42)

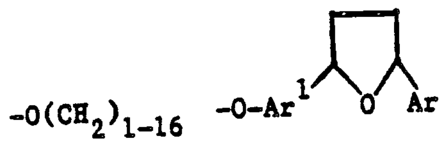

or

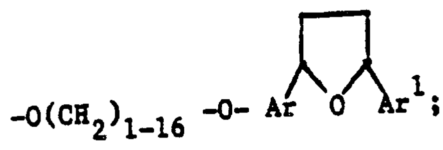

for example,

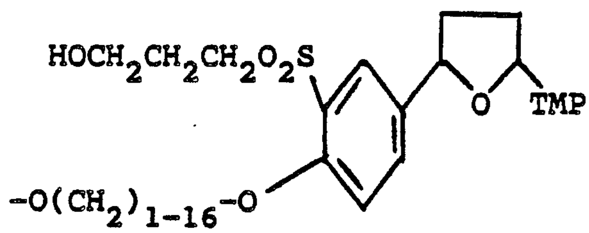

wherein TMP represents 3,4,5-trimethoxyphenyl;
43) heteroaryl as defined below especially pyrryl;
44) $-SO_2(CH_2)_{1-6}CONH_2$ such as $-SO_2CH_2CH_2CONH_2$;
45) $-(CH_2)_{1-6}CONR^2R^3$ such as $-CH_2CONH_2$;
46) $-(CH_2)_{1-6}NR^2SO_2R^3$ such as $-CH_2NHSO_2CH_3$;
47) $-(CH_2)_{1-6}OSO_2R^2$, e.g., $(CH_2)_6OSO_2CH_3$;
48) $-O(CH_2)_{1-6}-NR_2R_3$, e.g., $-OCH_2CH_2N(CH_3)_2$, and $-O(CH_2)_3N(CH_3)_2$;
49) $-CO-NR^2-CO-R^3$ such as $-CO-NH-CO-CH_3$;
50) $-S-CO-NR^2R^3$ such as $-S-CO-N(CH_3)_2$;
51) $-PO_3(CH_2CH_2N(CH_3)_2)$ $(C_2H_5)$,
52) $-S(O)_n(CH_2)_{0-5}CH_2CH(OR^2)R^3$ especially $-SO_2CH_2CH(OH)R^3$ such as $-SO_2CH_2CH(OH)CH_3$;
53) $-S(O)_n(CH_2)_{0-5}CH_2CH(OR^2)-(CO)R^3$ especially $-SO_2CH_2CH(OH)-(CO)C_2H_5$;
54) $-S(O)_n(CH_2)_{0-5}CH_2CH(OR^2)-(CO)NHR^3$; and
55) $-S(O)_n(CH_2)_{0-5}CH_2CH(OR^2)-(CO)OR^3$, for example, $-SO_2CH_2CH(OH)$ $(CO)OCH_3$.

(b) monoheteroaryl, di- or polyheteroaryl, or fused heteroaryl containing from 1 to 3 of any one or more of the heteroatoms N, S or O in each heteroaryl ring thereof and each ring may either be unsubstituted or substituted appropriately with a radical selected from a group consisting of $R^4$-$R^8$, for example, pyridyl, pyrryl, thienyl, isothiazolyl, imidazolyl, tetrazolyl, pyrazinyl, pyrimidyl, quinolyl, isoquinolyl, benzothienyl, isobenzofuryl, pyrazolyl, indolyl, purinyl, carbozolyl, and isoxazolyl and the like.
The following are the preferred heteroaryl groups:
(1) pyrryl or pyrryl substituted with $R^4$-$R^6$;
(2) furyl or furyl substituted with $R^4$-$R^6$;
(3) pyridyl or pyridyl substituted with $R^4$-$R^7$, such as 5,6-dimethoxypyridyl;
(4) thiophene or thiophene substituted with $R^4$-$R^6$;
(5) thiazolyl or thiazolyl substituted with $R^4$-$R^5$; or
(6) pyrimidyl or pyrimidyl substituted with $R^4$-$R^6$;

(c) heteroarylalkyl such as 2-pyridylmethyl, 2-thienylmethyl and 3-isothiazolylethyl;

(d) heterocycloalkyl e.g., 1,3-dioxacyclohex-4-yl, piperidino, morpholino, oxacyclopropyl, pyrrolidino, tetrazolo, benzothiazolo, imidazolidino, pyrazolidino, and piperazino; or

(e) heterocycloalkenyl such as pyrrolino, 2-imidazolino, 3-pyrazolino or isoindolino.

The compound of formula (I) can exist in the eight isomers as described in Scheme I. These various isomers bear a close relationship to the PAF antagonistic activity observed for the compounds within the scope of this invention.

Preferably, the PAF antagonists of this invention are of structural formula

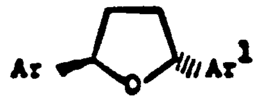

or an enantiomer thereof wherein Ar and $Ar^1$ are as previously defined.

Even more preferably, the compounds of this invention are of structural formula

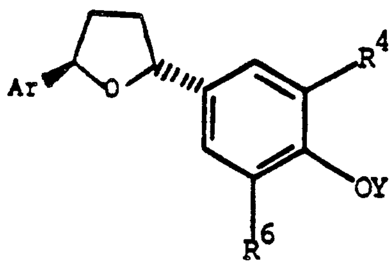

trans

wherein Ar, $R^4$, $R^6$ and Y are as previously defined.

B. Preparation of the compounds within the scope of the invention

The PAF antagonists of this invention have been prepared largely by stereospecific reactions from diaroylbutanes, bromo-ferulic acid derivatives, substituted phenyl vinyl ketones or styrene derivatives as indicated in the following schemes.

(1) Synthesis from reduction of diaroylbutanes, for example:

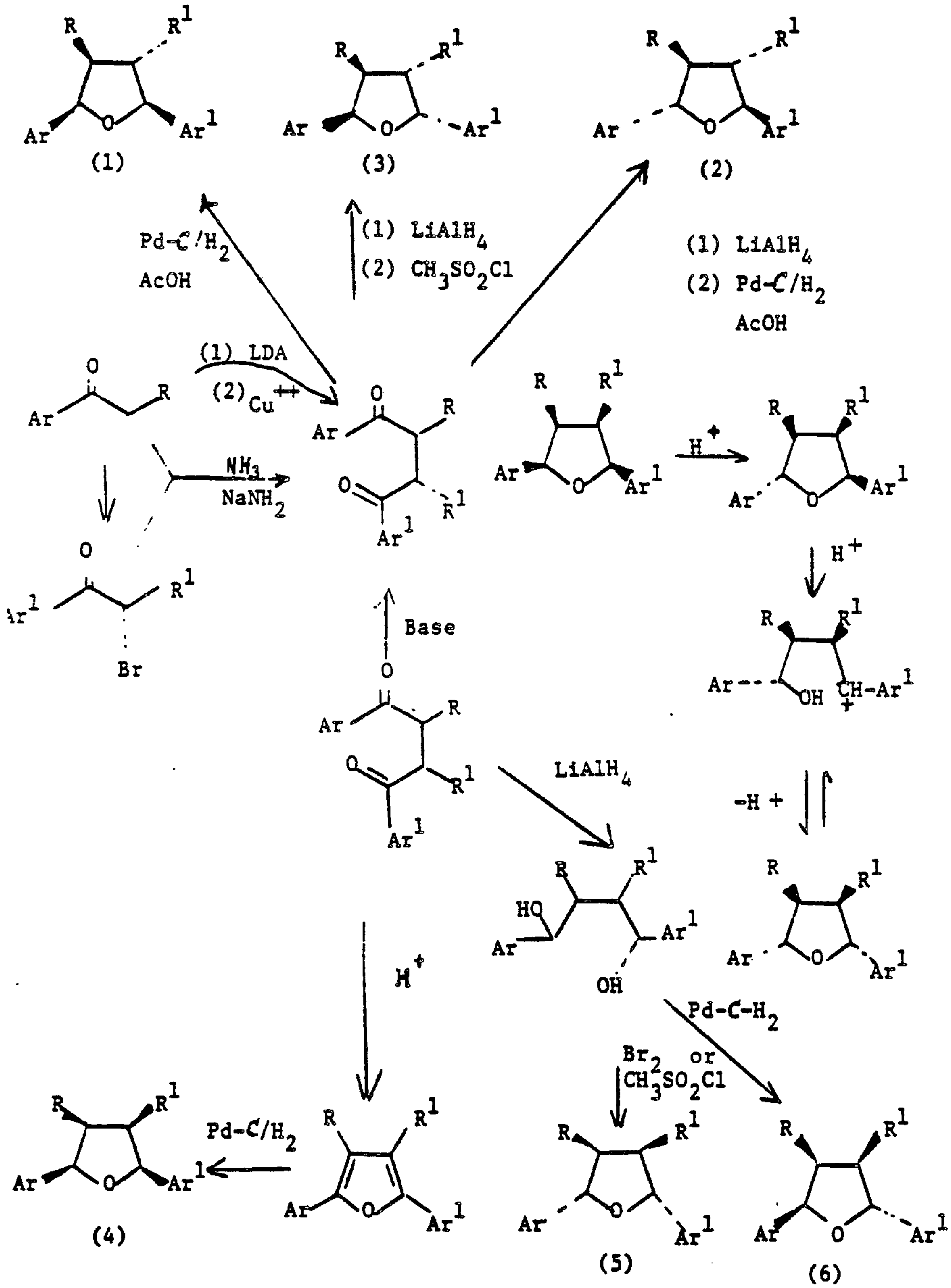


(2) Synthesis by oxidative coupling of bromo-ferulic acid derivatives, for example:

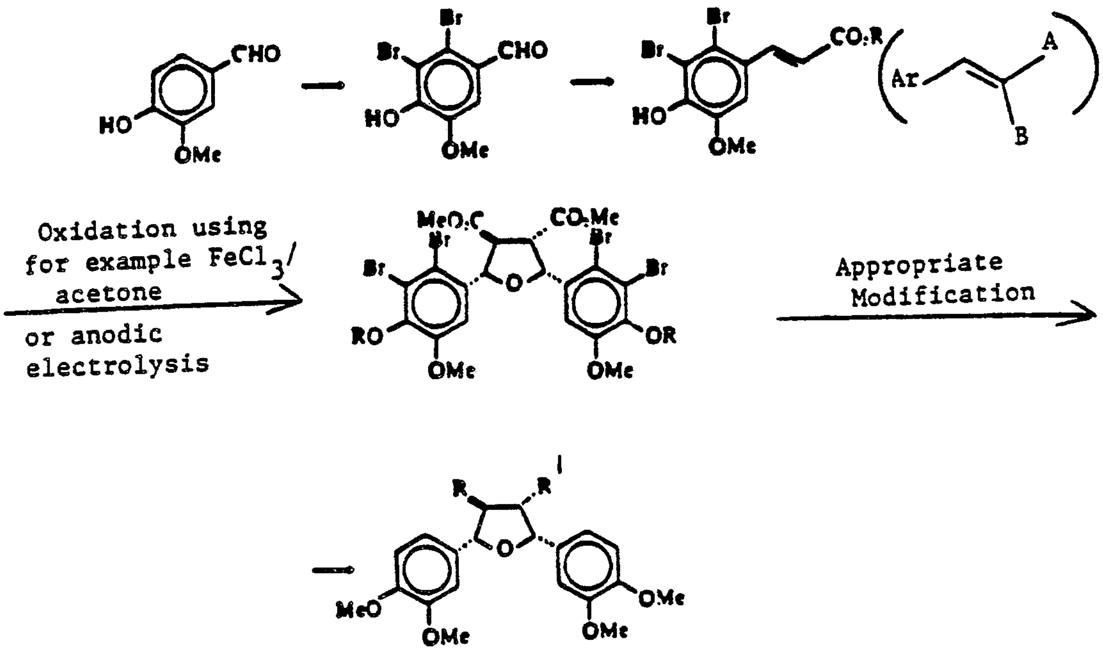


(3) Synthesis by acidic cleavage, for example:

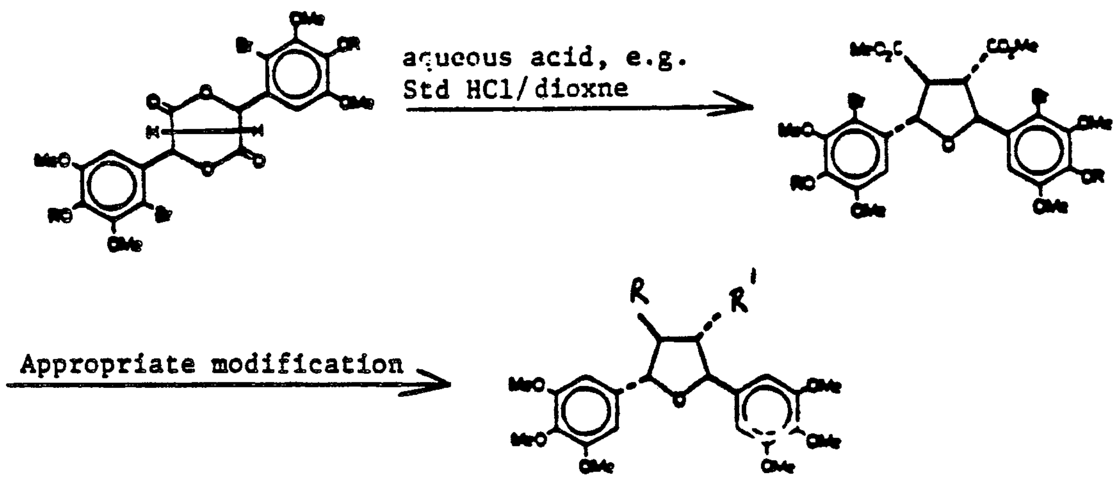


(4) Synthesis by oxidative dimerization using hydrogen peroxide catalyzed by peroxidase, for example:

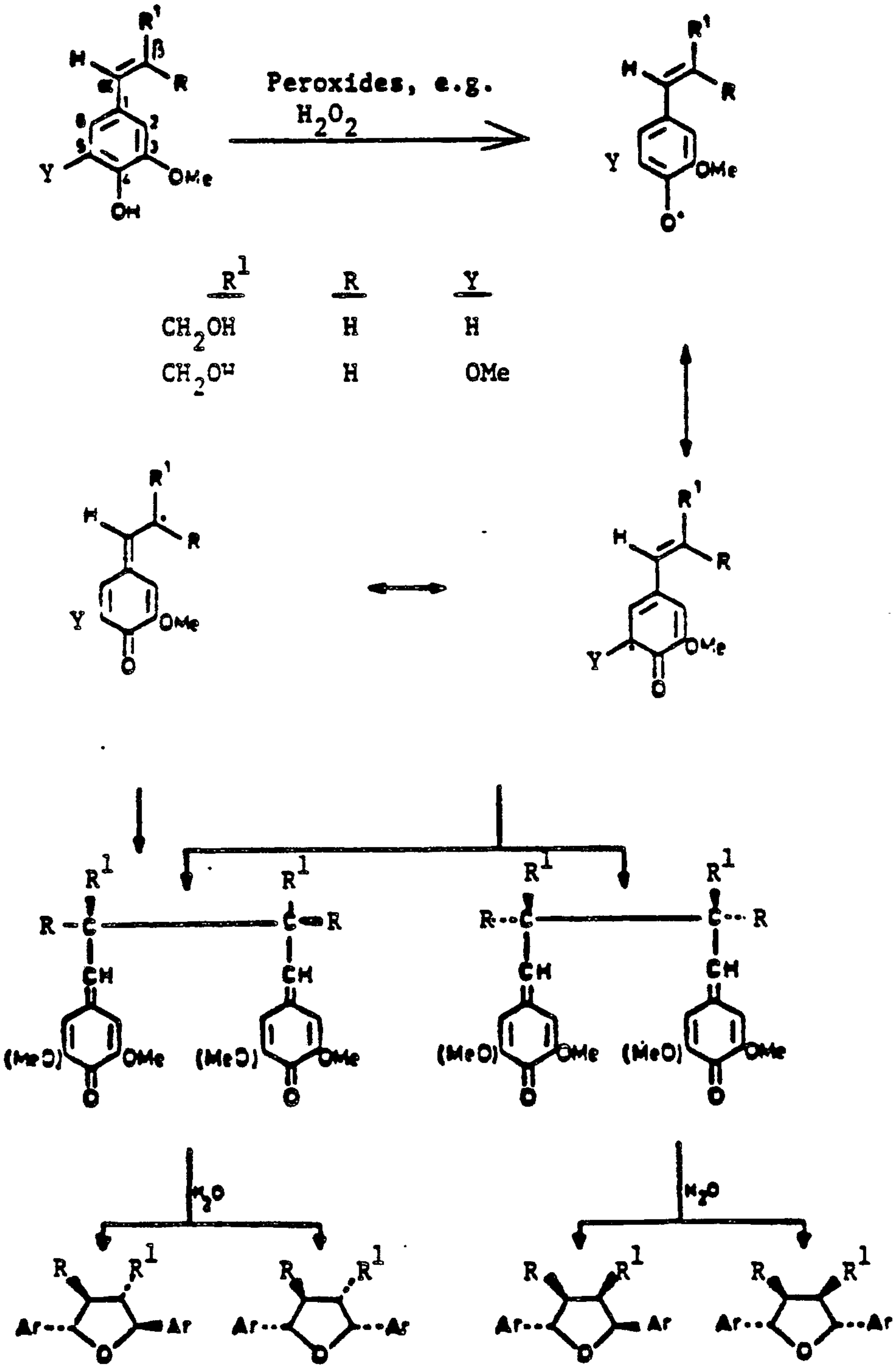


| $R^1$ | R | Y |
|---|---|---|
| $CH_2OH$ | H | H |
| $CH_2OH$ | H | OMe |

(5) Synthesis of unsymmetrical 2,5-diaryltetrahydrofurans:

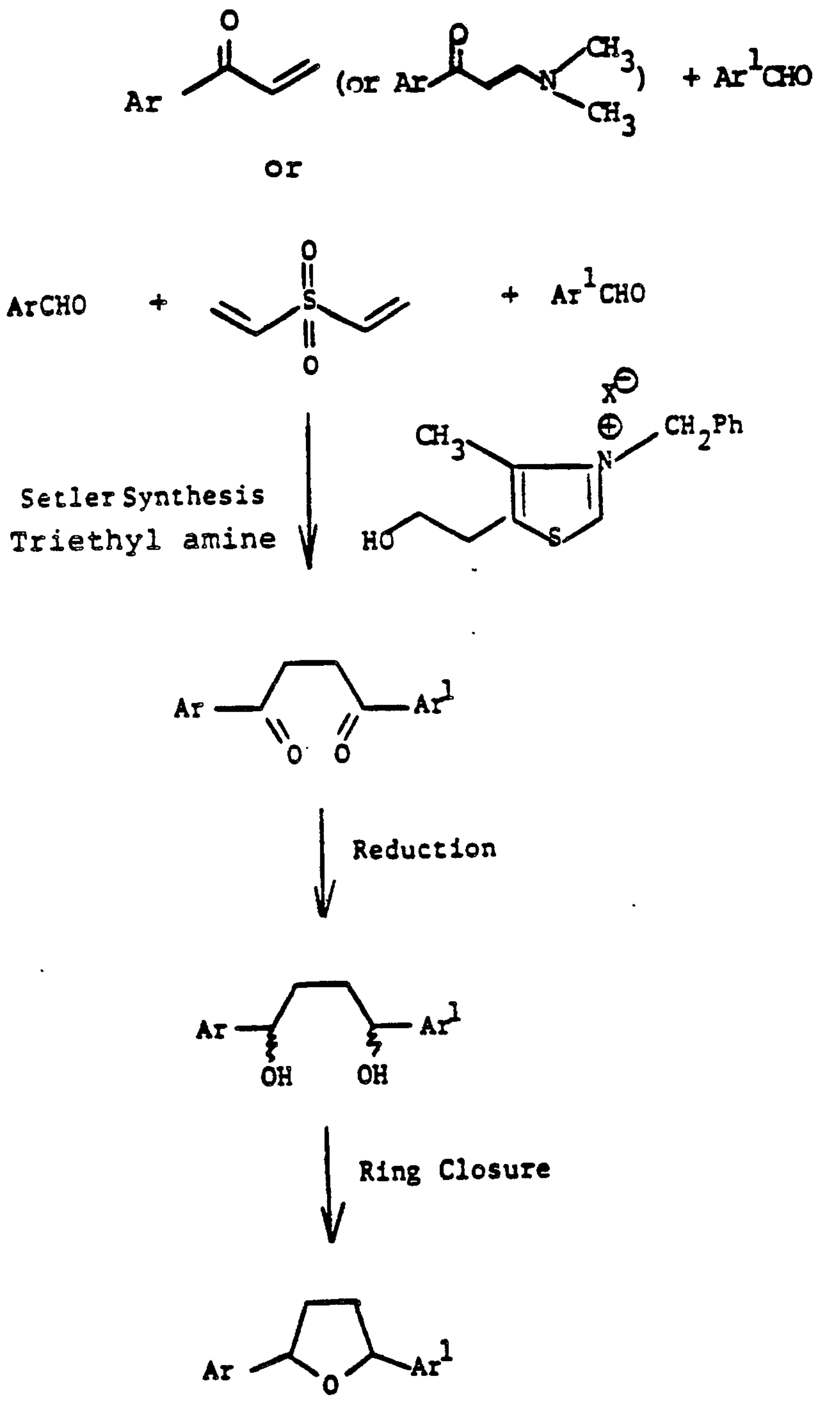


cis and trans-isomers

(6) Synthesis of Compound A

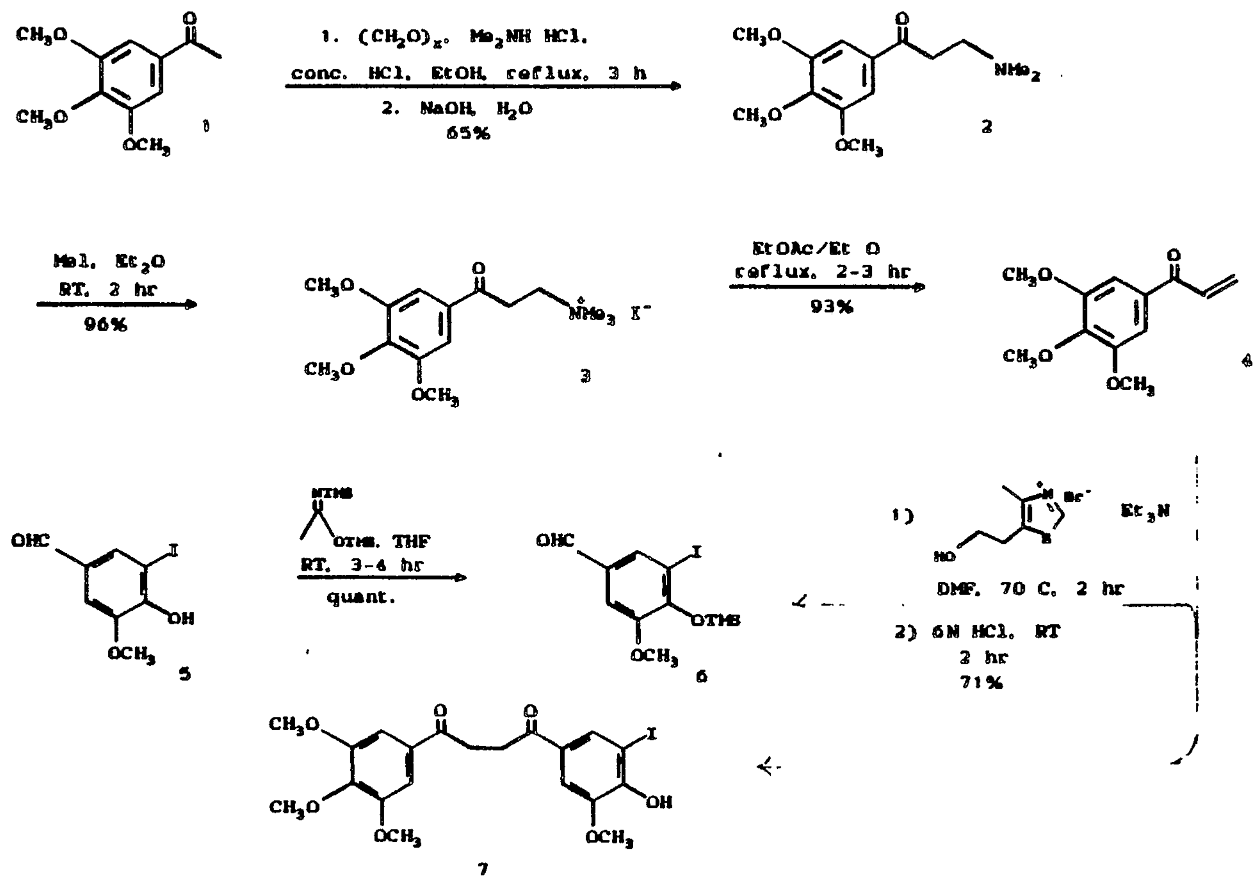

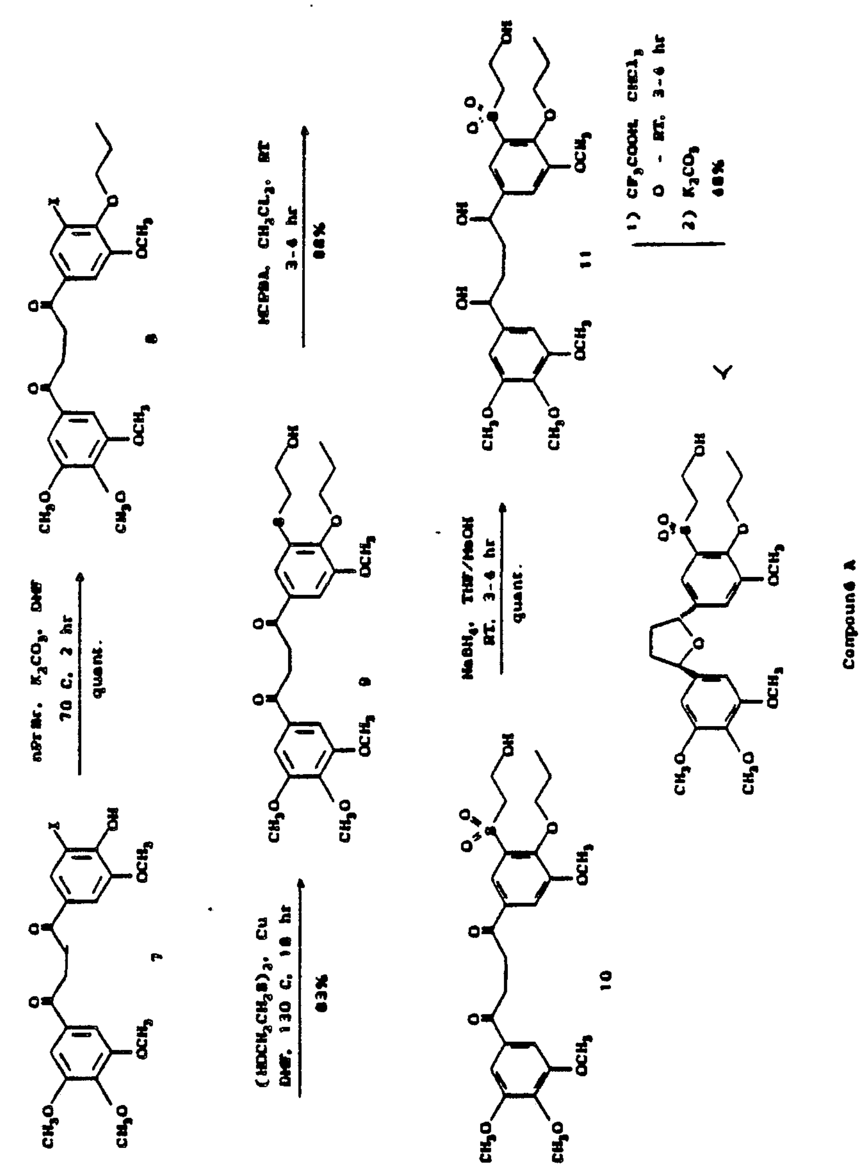


As shown above in schemes (1) to (5), most of the tetrahydrofuran PAF antagonists of this invention have been prepared from 1,4-diaryl-1,4-diketones by a two step reaction which involves reduction of the diketones to 1,4-diols followed by cyclization to the desired tetrahydrofurans.

The 1,4-diketones required to make the tetrahydrofurans were made by three different routes. In the first method, sodium, lithium, potassium or other metal generated from aryl ketones and a base such as sodium hydride, potassium hydride, sodium amide, n-butyl lithium, or lithium diisopropylamide were alkylated with α-bromo ketones or 2-iodoketones. This reaction could be carried out in various solvents such as anhydrous ether, tetrahydrofuran, dimethylformamide, dimethylsulfoxide or liquid ammonia at -78 °C to 50 °C. The second method of preparing 1,4-diketones involves the coupling of the metal enolates described above by using copper and iron salts such as cupric chloride, cupric triflate, ferric or ferrous chloride. A third method of preparing 1,4-diketones involves the reaction of an aryl aldehyde with an aryl vinyl ketone or the corresponding mannich base in the presence of a base such as triethylamine catalyzed by cyanide ion or thiazolium halides. This reaction could be done conveniently in solvents such as ethanol and dimethyl formamide at 25-80 °C.

Reduction of 1,4-diketones was carried out either catalytically by using hydrogen as the reducing agent or by using metal hydride reducing agents. In the later case, one can use the usual reducing agents such as lithium aluminum hydride in solvents such as ether or tetrahydrofuran or sodium borohydride in methanol, ethanol or similar solvents. These reductions could be carried out at -30 to 50 °C by stirring the substrate and the reducing agent dissolved in a solvent for 15 minutes to 24 hours. Alternatively, the 1,4-diketones could be reduced catalytically by using hydrogen and the usual catalysts such as palladium, platinum, rhodium or nickel in various solvents such as methanol, ethanol, tetrahydrofuran, acetic acid, ethyl acetate or benzene. Other acidic, basic or neutral low boiling solvents could also be used for the same purpose. The reaction mixture could be rendered acidic by using the usual mineral and organic acids such as hydrochloride, sulfuric, perchloric, trifluoroacetic or acetic acids. The reaction mixture could also be rendered basic by using the usual inorganic and organic bases such as sodium hydroxide, potassium hydroxide or triethylamine. The reduction could be carried out by stirring the substrate and the catalyst over hydrogen gas at 40-1500 p.s.i. for 15 minutes to 24 hours at 25-100 °C.

Cyclization of 1,4-diols to tetrahydrofurans was effected by stirring the diols with methane sulfonyl chloride-triethylamine, triphenylphosphine dibromide or trifluoroacetic acid in solvents such as methylene chloride, acetonitrile or chloroform. The reaction is conducted at -30 °C to 50 °C for 5 minutes to 4 hours. Alternatively, the 1,4-diols could be cyclized to tetrahydrofurans by heating the diols at 0-50 °C above their melting points in the presence of catalytic amounts (.01 -4%) of palladium, platinum and/or copper salts. The preferred metal salts are platinum chloride, platinum acetate, copper acetate and copper nitrate.

The tetrahydrofuran PAF antagonists of this invention could also be made from aryl propene or cinnamic acid derivatives either by chemical oxidative coupling reactions, or by using ferric chloride in solvents such as acetone, or enzymatically by using peroxidases such as horseradish peroxidase in solvents such as aqueous acetone. The above reactions are usually carried out at 0-40 °C for 1-14 days.

C. Utility of the compounds within the scope of the invention

This invention also relates to a method of treatment for patients (or mammalian animals raised in the dairy, meat, or fur industries or as pets) suffering from disorders or diseases which can be attributed to PAF as previously described, and more specifically, a method of treatment involving the administration of the PAF antagonists of formula (I) as the active constituents.

Accordingly, the compounds of Formula (I) can be used among other things to reduce pain and inflammation, to correct respiratory, cardiovascular, and intravascular alterations or disorders, and to regulate the activation or coagulation of platelets, to correct hypotension during shock, the pathogenesis of immune complex deposition and smooth muscle contractions.

For the treatment of inflammation such as rhumatoid arthritis, osteoarthritis, and eye inflammation, cardio-vascular disorder, asthma, shock syndrome or other diseases mediated by the PAF, the compounds of Formula (I) may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle, sheep, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Patents 4,256,108; 4,166,452; and 4,265,874 to form osmotic therapeutic tablets for control release.

Formulaticns for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oil suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The compounds of Formula (I) may also be administered in the form of suppositories for rectal administration of the drug. These compositions can be prepared by mixing the drug with a suitable nonirritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compounds of Formula (I) are employed.

Dosage levels of the order of from about 0.1 mg to about 140 mg per kilogram of body weight per day are useful in the treatment of the above-indicated conditions (about 0.5 mg to about 7 gms. per patient per day). For example, inflammation may be effectively treated by the administration of from about 0.01 to 50 mg of the compound per kilogram of body weight per day (about 1.0 mg to about 3.5 gms per patient per day).

The amount of active ingredient that may be combined with the carrier materials to produce a single dosage form will vary depending upon the host treated and the particular mode of administration. For example, a formulation intended for the oral administration of humans may contain from 0.5 mg to 5 gm of active agent compounded with an appropriate and convenient amount of carrier material which may vary from about 5 to about 95 percent of the total composition. Dosage unit forms will generally contain between from about 1 mg to about 500 mg of an active ingredient.

It will be understood, however, that the specific dose level for any particular patient will depend upon a

variety of factors including the activity of the specific compound employed, the age, body weight, general health, sex, diet, time of administration, route of administration, rate of excretion, drug combination and the severity of the particular disease undergoing therapy.

D. Bioassay Results Supporting the utility of the compounds of the present invention

It has been found that the compounds of formula (I) exhibit antagonistic activities with respect to PAF:

In Vitro Assay:

In vitro, the compounds of formula (I) inhibit PAF-induced functions in both the cellular and tissue levels by changing the PAF binding to its specific receptor site. The ability of a compound of formula (I) to inhibit the PAF binding to its specific receptor binding site on rabbit or human platelet or PMN plasma membranes was measured by an assay recently developed by us.

The inhibition of $H^3$-PAF binding to the human or rabbit platelet or PMN plasma membrane by a PAF antagonist of Formula (I) was determined by a method employing isotopic labeling and filtration techniques. Generally, a series of Tris-buffered solutions of the selected antagonist at predetermined concentrations were prepared. Each of these solutions contains 1 pmole of $^3$H-PAF, a known amount of the test antagonist, and a sufficient amount of the pH 7.5 Tris-buffer solution (10mM Tris, 0.25% bovine serum albumin, and 150 mM NaCl per ml water) to make the final volume of 1 ml. After adding into a set of test tubes each with 100 $\mu$g of the platelet plasma membrane suspension (S.B. Hwang, et al., Biochemistry, Vol. 22, pp. 4756-4763, 1983) and one of the Tris-buffer solutions described above, the resulting mixture in each test tube was incubated at 0°C for about one hour or until the reaction was complete. Two control samples, one of which ($C_1$)contains all the ingredients described above except the antagonist and the other ($C_2$) contains $C_1$ plus a 1000-fold excess of unlabeled PAF, were also prepared and incubated simultaneously with the test samples. After the incubation was completed, the contents of each test tube were filtered under vacuo through a Whatman GF/C fiberglass filter and the residue washed rapidly several times with a total of 20 ml cold (0°-5°C) Tris-buffer solution. Each washed residue was then suspended in 10 ml scintillation solution (Aquasol 2, New England Nuclear, Connecticut) and the radioactivity was counted in a Packard Tri-Carb 460CD Liquid Scintillation system. Defining the counts from a test sample as "Total binding with antagonist"; the counts from the control sample $C_1$, as "Total binding $C_1$"; and the counts from the control sample $C_2$ as non-specific binding $C_2$", the percent inhibition of each test antagonist can be determined by the following equation:

$$\%\ \text{Inhibition} = \frac{(\text{Total binding } C_1) - \left(\begin{array}{l}\text{Total binding}\\ \text{with antagonist}\end{array}\right)}{\text{Specific binding}} \times 100$$

$$\text{Specific binding} = (\text{Total binding } C_1) - (\text{non-specific binding } C_2)$$

From our observation, compounds of formula (I) inhibit in vitro PAF-induced platelet aggregation (rabbit or human platelets); PAF-induced guinea pig peritoneal PMN (polymorphonuclear leukocytes) aggregation; PAF-induced human PMN secretion; and PAF-induced guinea pig smooth muscle contraction although they are not $H_2$-receptor antagonists. They are also shown in these inhibition studies to be highly specific to PAF. For example, they do not inhibit the binding of $H_1$ antagonist ($^3$H-pyrilamine) to guinea pig brain membrane, nor do they inhibit the binding of a cholecystokinin (CCK) receptor based on an assay on isolated rat pancreas membrane. Furthermore, they affect no or only minute inhibition on the histamine-induced ileum contraction from guinea pigs.

Results from the In Vitro assay

The antagonistic activity of the compounds of structural formula (I) is summarized in the following tables

(A), (B), (C), and (D):

Table (A)

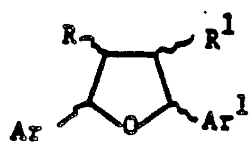

| R | $R^1$ | Ar | $Ar^1$ | isomer | dose(μM) | Rabbit platelet membrane % inhibition |
|---|---|---|---|---|---|---|
| H | H | 3,4-dimethoxy-phenyl | napthyl | trans | 5 | 80 |
| H | H | 3-methoxy-4-hydroxyphenyl | same as Ar | trans | 5<br>1<br>.5<br>.1 | 100<br>74<br>54<br>4 |
| H | H | 3-dimethylamino-4-chlorophenyl | " | trans | 5 | 47 |
| " | " | " | " | cis | 5 | 57 |
| H | H | 3-ethyl-4-methoxyphenyl | " | trans | 1 | 17 |
| H | H | 3-methoxy-4-benzyloxyphenyl | " | " | 1<br>1 | 12<br>4 |
| H | H | 3-methoxy-4-hydroxyphenyl | " | " | 5 | 94 |
| H | H | 3-methoxy-4-methylthio-phenyl | " | " | 5 | 40 |

| R | $R^1$ | Ar | $Ar^1$ | isomer | dose(µM) | Rabbit platelet membrane % inhibition |
|---|---|---|---|---|---|---|
| " | " | " | " | cis | 5 | 61 |
| H | H | 3-dimethylamino-4-methoxyphenyl | " | cis; trans 1:1 | 5<br>1 | 96<br>4 |
| H | H | 3-propoxy-4-methoxyphenyl | " | trans | 5<br>1<br>0.5<br>0.1 | 85<br>63<br>54<br>30 |
| H | H | 3-ethoxy-4-methoxyphenyl | " | " | 5<br>1<br>0.5<br>0.1 | 80<br>63<br>48<br>8 |
| H | H | 3,4,5-tri-methoxyphenyl | same as Ar | " | 1<br>.3<br>.1<br>.03<br>.01 | 80<br>77<br>61<br>40<br>25 |
| " | " | | " | cis | 5<br>1 | 43<br>0 |
| H | H | 3-ethyl-4-methoxyphenyl | " | cis | 5 | 31 |

| R | $R^1$ | Ar | $Ar^1$ | isomer | dose(μM) | Rabbit platelet membrane % inhibition |
|---|---|---|---|---|---|---|
| | | | | | 5 | 86 |
| H | H | 3,4-dimethoxy-phenyl | " | trans | 3 | 87 |
| | | | | | 1 | 89 |
| | | | | | .3 | 79 |
| | | | | | .1 | 63 |
| | | | | | .03 | 24 |
| | | | | | .01 | 21 |
| | | | | | 1 | 65 |
| " | " | " | " | cis | .3 | 57 |
| | | | | | .1 | 17 |
| " | " | 3,4,5-trimethoxy phenyl | 5,6-di-methoxy-3-pyridyl | trans | 5 | 96 |
| | | | | | 1 | 88 |
| | | | | | .3 | 85 |
| | | | | | .1 | 64 |
| | | | | | .03 | 38 |
| " | " | " | 5,6-di-methoxy-3-pyridyl | cis | 5 | 69 |
| | | | | | 1 | 39 |
| | | | | | .3 | 20 |
| " | " | " | 2,6-di-methoxy-4-pyridyl | trans | 5 | 86 |
| | | | | | 1 | 70 |
| | | | | | .3 | 48 |
| " | " | " | 5,6-di-methoxy-2-pyridyl | " | 5 | 96 |
| | | | | | 1 | 85 |
| | | | | | .3 | 66 |
| " | " | " | 6-methoxy-3-pyridyl | " | 5 | 64 |
| | | | | | 1 | 25 |

| R | $R^1$ | Ar | $Ar^1$ | isomer | dose(μM) | Rabbit platelet membrane % inhibition |
|---|---|---|---|---|---|---|
| H | H | 2-thienyl | 2-thienyl | trans | 5 | 29 |
| " | " | 5,6-dimethoxy-3-pyridyl | Same as Ar | " | 5 | 75 |
| | | | | | 1 | 46 |
| | | | | | .3 | 30 |

Table (B)

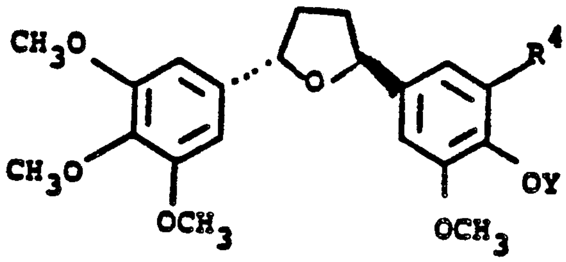

| $R^4$ | Y | Rabbit platelet membrane inhibition $IC_{50}$ (nM) |
|---|---|---|
| CN | $CH_2CH{=}CH_2$ | 46.0 |
| CN | $CH_2CH_2CH_3$ | 8.7 |
| I | $CH_2CH{=}CH_2$ | 13.0 |
| $OCH_3$ | $CH_2$ 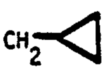 | 8.3 |
| $OCH_3$ | $CH_2CH{=}CH_2$ | 58.0 |
| $SCH_3$ | $CH_2CH{=}CH_2$ | 23.0 |
| $SCH_3$ | $CH_2CH_2CH_3$ | 7.0 |
| $SOCH_3$ | $CH_2CH{=}CH_2$ | 10.0 |
| $SO_2CH_3$ | $CH_2CH{=}CH_2$ | 2.0 |
| $SO_2CH_3$ | $CH_2CH_2CH_3$ | 1.0 |
| $NO_2$ | $CH_2CH{=}CH_2$ | 23.0 |
| $NO_2$ | $CH_3$ | 23.0 |
| $NO_2$ | $CH_2CH_2CH_3$ | 4.4 |
| $NO_2$ | $CH_2CH_2CH_2CH_3$ | 3.0 |
| $NO_2$ | $CH_2CH_2CH_2CH_2CH_3$ | 1.5 |
| $NO_2$ | $CH_2CO_2CH_2CH_3$ | 23.0 |
| $NO_2$ | $CH_2$ 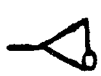 | 28.0 |

| $R^4$ | Y | $IC_{50}$ (nM) |
|---|---|---|
| $NH_2$ | $CH_2CH{=}CH_2$ | 80.0 |
| $NHCH_2CO_2$ | $EtCH_2CH{=}CH_2$ | 58.0 |
| $NH_2$ | $CH_2CH_2CH_3$ | 30.0 |
| $N(CH_3)_2$ | $CH_2CH = CH_2$ | 12.0 |
| $^+N(CH_3)_3I^-$ | $CH_2CH{=}CH_2$ | 140.0 |
| $NHSO_2CH_3$ | $CH_2CH{=}CH_2$ | 10.0 |
| $NHSO_2CH_3$ | $CH_2CH_2CH_3$ | 4.0 |
| $N(CH_3)SO_2CH_3$ | $CH_2CH{=}CH_2$ | 20.0 |
| $NHCOCH_3$ | $CH_2CH_2CH_3$ | 33.0 |
| $CONH_2$ | $CH_2CH_2CH_3$ | 23.0 |

Table (C)

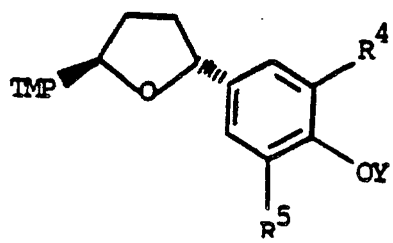

| $R^4$ | Y | $R^5$ | Dose (nM) | % Inhibition of $^3$H-PAF binding (Human platelet) |
|---|---|---|---|---|
| $SO_2CH_3$ | $(CH_2)_3CH(F)CH_3$ | $OCH_3$ | 30 | 46 |
| $SO_2CH_3$ | $CH_2CF_2CF_3$ | $OCH_3$ | 30 | 42 |
| $SO_2CH_3$ | $(CH_2)_6F$ | $OCH_3$ | 30 | 41 |
| $SO_2CH_2CH_2CH_3$ | $(CH_2)_3Cl$ | $OCH_3$ | 30 | 76 |
| | | | 3 | 24 |
| $SO_2CH_2CH_2CH_3$ | $CH_2CH_2Br$ | $OCH_3$ | 30 | 71 |
| | | | 3 | 31 |
| $SO_2CH_2CH_2CH_3$ | $(CH_2)_4Br$ | $OCH_3$ | 30 | 93 |
| | | | 3 | 46 |
| $SO_2(CH_2)_3OH$ | $C_2H_5$ | $OCH_3$ | 30 | 59 |
| | | | 3 | 11 |
| $SO_2CH_3$ | $(CH_2)_6OH$ | $OCH_3$ | 30 | 52 |
| | | | 3 | 23 |
| $SO_2CH_3$ | $(CH_2)_4CH(OH)CH_3$ | $OCH_3$ | 30 | 42 |
| | | | 3 | 20 |
| $SO_2CH_3$ | $CH_2CH_2OCH_2CH_3$ | $OCH_3$ | 30 | 35 |
| $SO_2(CH_2)_2OC_2H_5$ | $C_3H_7$ | $OCH_3$ | 30 | 85 |
| | | | 3 | 44 |
| $SO_2(CH_2)_2COOCH_3$ | $C_2H_5$ | $OCH_3$ | 30 | 36 |
| $SO_2C_3H_7$ | $C_2H_5$ | $OCONHCH_3$ | 30 | 66 |
| | | | 3 | 14 |
| CO-(1-imidazolyl) | $C_2H_5$ | $OCH_3$ | 30 | 24 |

| $R^4$ | Y | $R^5$ | Dose (nM) | % Inhibition of $^3H$-PAF binding (Human platelet) |
|---|---|---|---|---|
| CO-(2-thiazole) | $CH_2H_5$ | $OCH_3$ | 30 | 48 |
| | | | 3 | 13 |
| $SO_2C_3H_7$ | $CH_2$-(2-pyridyl) | $OCH_3$ | 30 | 82 |
| | | | 3 | 32 |
| $SO_2C_3H_7$ | $(CH_2)_3$-(3-pyridyl) | $OCH_3$ | 30 | 68 |
| | | | 3 | 25 |
| $SO_2C_3H_7$ | $C_2H_5$ | $OCH_2$-(2-pyridyl) | 30 | 85 |
| | | | 3 | 48 |
| $-CO-NHCH_2CH_2-$ (1-morpholinyl) | $C_2H_5$ | $OCH_3$ | 30 | 21 |
| $SO_2C_3H_7$ | $(CH_2)_4$-(1-morpholinyl) | $OCH_3$ | 30 | 40 |
| | | | 3 | 11 |
| $SO_2CH(CH_3)_2$ | $C_3H_7$ | $OCH_3$ | 30 | 63 |
| | | | 3 | 14 |
| $SO_2-CH_3$ | $CH(CH_3)CH_2CH_3$ | $OCH_3$ | 30 | 44 |
| $SO_2-CH_3$ | $CH_2CH(CH_3)_2$ | $OCH_3$ | 30 | 42 |
| $SO_2-CH_3$ | t-Bu | $OCH_3$ | 30 | 28 |
| $SO_2-CH_3$ | $CH(C_5H_{11})_2$ | $OCH_3$ | 30 | 57 |
| | | | 3 | 18 |
| $SO_2-CH_3$ | $(CH_2)_4CH=CH_2$ | $OCH_3$ | 30 | 73 |
| | | | 3 | 27 |
| $SO_2(CH_2)_2CH=CH_2$ | $C_2H_5$ | $OCH_3$ | 30 | 85 |
| | | | 3 | 48 |
| $SO_2(CH_2)_2C\equiv CH$ | $C_2H_5$ | $OCH_3$ | 30 | 89 |
| | | | 3 | 54 |
| $SO_2-CH_3$ | $(CH_2)_3$-cyclohexyl | $OCH_3$ | 30 | 67 |
| | | | 3 | 24 |
| $SO_2-CH_3$ | $(CH_2)_3$-cyclopentyl | $OCH_3$ | 30 | 75 |
| | | | 3 | 29 |

| $R^4$ | Y | $R^5$ | Dose (nM) | % Inhibition of $^3$H-PAF binding (Human platelet) |
|---|---|---|---|---|
| $SO_2-CH_2C_6H_5$ | $C_2H_5$ | $OCH_3$ | 30 | 88 |
| | | | 3 | 51 |
| $SO_2CH_2C_6H_5$ | $C_3H_7$ | $OCH_3$ | 30 | 55 |
| $SO_2CH_3$ | $(CH_2)_3-C_6H_5$ | $OCH_3$ | 30 | 53 |
| | | | 3 | 11 |
| $SO_2-CH_3$ | $(CH_2)_6C_6H_5$ | $OCH_3$ | 30 | 66 |
| | | | 3 | 15 |
| $SO_2C_3H_7$ | $(CH_2)_3C_6H_5$ | $OCH_3$ | 30 | 95 |
| | | | 3 | 56 |
| CO-NH-(p-F-$C_6H_5$) | $C_2H_5$ | $OCH_3$ | 30 | 49 |
| | | | 3 | 12 |
| $SO_2-CH_2C_6H_5$ | $CH_2C_6H_5$ | $OCH_3$ | 30 | 39 |
| | | | 3 | 24 |
| $SO_2C_3H_7$ | $C_2H_5$ | $OCH_2$-(p-F-$C_6H_5$) | 30 | 82 |
| | | | 3 | 36 |
| N-pyrryl | $C_3H_7$ | $OCH_3$ | 30 | 13 |
| $SO_2(CH_2)_2CONH_2$ | $C_2H_5$ | $OCH_3$ | 30 | 13 |
| | | | 3 | 14 |
| $SO_2-CH_3$ | $(CH_2)_6OSO_2CH_3$ | $OCH_3$ | 30 | 24 |
| $CO-NH-(CH_2)_2N-(CH_3)_2$ | $C_2H_5$ | $OCH_3$ | 30 | 7 |
| | | | 3 | 5 |
| $SO_2-CH_3$ | $(CH_2)_6NH_2$ | $OCH_3$ | 30 | 33 |
| $SO_2-CH_3$ | $C_2H_5$ | $(CH_2)_2N(CH_3)_2$ | 30 | 48 |
| $SO_2-C_3H_7$ | $(CH_2)_4N^+(CH_3)_3Br^-$ | $OCH_3$ | 30 | 10 |
| $SO_2-CH_3$ | $(CH_2)_6N_3$ | $OCH_3$ | 30 | 59 |

| $R^4$ | Y | $R^5$ | Dose (nM) | % Inhibition of $^3H$-PAF binding (Human platelet) |
|---|---|---|---|---|
| $SO_2-C_3H_7$ | $(CH_2)_5CN$ | $OCH_3$ | 30 | 86 |
| | | | 3 | 51 |
| $CH_2SCH_3$ | $C_3H_7$ | $OCH_3$ | 30 | 18 |
| $SO_2C_3H_7$ | $(CH_2)_2SCH_3$ | $OCH_3$ | 30 | 90 |
| | | | 3 | 61 |
| $SO_2C_3H_7$ | $(CH_2)_2SC_6H_5$ | $OCH_3$ | 30 | 93 |
| | | | 3 | 41 |
| $SO_2C_3H_7$ | $(CH_2)_2SOCH_3$ | $OCH_3$ | 30 | 34 |
| $SO_2-C_3H_7$ | $(CH_2)_2SO_2CH_3$ | $OCH_3$ | 30 | 40 |
| $PO(OH)(OC_2H_5)$ | $C_3H_7$ | $OCH_3$ | 30 | 2 |
| | | | 3 | 1 |
| $PO-OCH_2CH_2N(CH_3)_2$ | $C_3H_7$ | $OCH_3$ | 30 | 25 |
| $OC_2H_5$ | | | 3 | 19 |

TABLE (D)

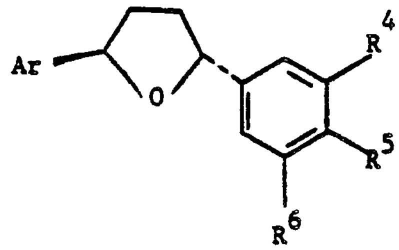

| Ar | $R^4$ | $R^5$ | $R^6$ | dose (mM) | % Inhibition of 3H-PAF Binding: Rabbit Platelet | Human Platelet |
|---|---|---|---|---|---|---|
| TMP | $CF_3$ | $OC_3H_7$ | $OCH_3$ | 30 | 15 | - |
| TMP | $CH_2OH$ | $OC_3H_7$ | $OCH_3$ | 30 | 56 | - |
| TMP | $CH_2OC_2H_5$ | $OC_3H_7$ | $OCH_3$ | 30 | 72 | - |
| TMP | $CH_2OCOCH_3$ | $OC_3H_7$ | $OCH_3$ | 30 | 42 | - |
| 3,5-dimethoxy-4-(Ph*CH($OCH_3$)-COO)-phenyl | $SO_2CH_3$ | $OC_3H_7$ | $OCH_3$ | 30 | 5 | - |
| TMP | Br | $OCH_2Ph$ | $OCH_3$ | 30 | 31 | - |
| TMP | $SO_2CH_2CH_2CONH_2$ | $OC_2H_5$ | $OCH_3$ | 30 | - | 13 |
| TMP | $CH_2NHSO_2CH_3$ | $OC_3H_7$ | $OCH_3$ | 30 | 63 | - |
| TMP | $CH_2NH_2$ | $OC_3H_7$ | $OCH_3$ | 30 | 18 | - |

| Ar | $R^4$ | $R^5$ | $R^6$ | dose (mM) | % Inhibition of $^3$H-PAF Binding Rabbit Platelet | Human Platelet |
|---|---|---|---|---|---|---|
| TMP | $CH_2NHCOCH_3$ | $OC_3H_7$ | $OCH_3$ | 30 | 16 | - |
| TMP | $CONHCOCH_3$ | $OC_3H_7$ | $OCH_3$ | 30 | 66 | - |
| TMP | $N_3$ | $OCH_2CH{=}CH_2$ | $OCH_3$ | 30 | 10 | - |
| TMP | $CH_2CN$ | $OC_3H_7$ | $OCH_3$ | 30 | 70 | - |
| TMP | $CH_2SCH_3$ | $OCH_2CH{=}CH_2$ | $OCH_3$ | 100 | 57 | - |
| TMP | $CH_2SOCH_3$ | $OC_3H_7$ | $OCH_3$ | 30 | 12 | - |
| TMP | $CH_2SO_2CH_3$ | $OC_3H_7$ | $OCH_3$ | 30 | 31 | - |
| TMP | $SO_2$-$C_3H_7$ | $SCON(CH_3)_2$ | $OCH_3$ | 30 | - | 18 |
| 3-(2,3-di-methoxypyridinyl) | $SO_2CH_3$ | $OCH_2CH_3$ | $OCH_3$ | 30 | - | 16 |
| 3-(2,3-di-methoxypyridinyl) | $SO_2$-$C_3H_7$ | $OCH_2CH_3$ | $OCH_3$ | 30<br>3 | -<br>- | 88<br>38 |

## EXAMPLE 1

### 2,5-Bis(3,4-Dimethoxyphenyl)tetrahydrofuran

Step A: Preparation of 1,2-bis(3,4-dimethoxybenzoyl)ethane

In a 500 ml flask equipped with a stirrer and $N_2$, LDA was prepared from 20 ml THF, 10.1 g diisopropylamine and 62 ml 1.7M n-butyl lithium at -10° C. The temperature was dropped to -40° C and then 18 g of 3,4-dimethoxyacetophene in 40 ml THF was added. After 1 hour, 13 g of anhydrous $CuCl_2$ in 150 ml DMF was added and stirring continued overnight.

500 Ml of 1N HCl was added and the resulting precipitate collected by filtration. The precipitate was dissolved in methylene chloride and filtered through a bed of silica gel. Evaporation followed by crystallization from ethyl acetate gave 4.5 g 1,2-bis(3,4-dimethoxybenzoyl)ethane as a white solid, m.p. 181-2° C NMR ($CDCl_3$): $\delta$ 3.40 (4H, s,

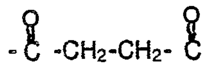 3.92 (12 H,s, 4 x $OCH_3$),6.8-7.74 (6H, ArH).

Step B: Preparation of 2,5-bis(3,4-dimethoxyphenyl)tetrahydrofuran

3.0 g of 1,2-bis(3,4-dimethoxybenzoyl)ethane was reduced with 350 mg of lithium aluminum hydride in 50 ml THF at 0° C for 1 hour and ambient temperature for 3 hours. After the usual workup, 2.8 g of white solid diol was recovered and dissolved in 50 ml of methylene chloride. The solution at 0° C was treated with 1.2 g of triethylamine followed by 1.2 g of methane sulfonyl chloride (or cyclization with any acid such as hydrochloric acid or trifloro acetic acid) and stirred under $N_2$ until the starting diol disappears by TLC. At this point, 200 ml of ether was added and the organic layer washed with water, 3N HCl, 10% NaOH, water and dried over anhydrous sodium sulfate. The residue obtained by evaporation was chromatographed to recover the major spot (silica gel, ethyl acetate-hexane 30:70). Crystallization from 50 ml methylene chloride and 100 ml of hexane evaporated to a final volume of 100 ml gave 0.6 g of trans- 2,5-bis (3,4-dimethoxyphenyl)tetrahydrofuran, NMR ($CDCl_3$): δ1.9-2.5 (4H, m, 3-H, 4-H) 3.85 and 3.90 (each s, 6H, 2 x $OCH_3$), 5.24 (2H, t, J=6.6 Hz, 2-, 5-H), and 6.8-7.0 (6H, m, ArH), m.p. 114-115° C. The second and third crops gave 1.1 g of crystalline product which contains a mixture of cis and trans isomers. Separation of the isomers was made on Partisil-10 column using hexane (80%), ethylacetate (20%) as eluting solvent. The NMR of the cis isomer (m.p. 92-96° C) is shown below.
NMR ( $CDCl_3$): δ1.9-2.5 (4H,m,3H,4-H), 3.88 and 3.83 (each s, 6H, 2 x $OCH_3$), 5.04 (2H, t, 6.7 Hz, 2-,5-H), and 6.8-7.0 (6H,m,Ar-H)

## EXAMPLE 2

Following synthetic schemes (2)-(4) as described at pages 8-9 of this specification, there are prepared the compounds as shown in the following tables:

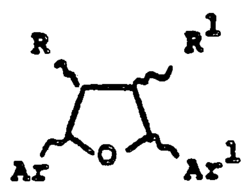

| R | $R^1$ | Ar | $Ar^1$ |
|---|---|---|---|
| H | $OCH_3$ | 3,4-dimethoxy-phenyl | Same as Ar |
| $OCH_3$ | $OCH_3$ | 4-methoxy-phenyl | Same as Ar |
| F | H | 3-dimethylamino-4-chlorophenyl | Same as Ar |
| F | F | 3,4-difluoro-phenyl | Same as Ar |

## EXAMPLE 3

### 2-(3,4-Dimethoxyphenyl)-5-(2-naphthyl)tetrahydrofuran

Step A: Preparation of 1-(3,4-dimethoxyphenyl)-4-(2-naphthyl)butan-1,4-dione

Sodium acetate (2.38 g), 3-benzyl-4-methyl-5-(2-hydroxyethyl)thiazolium chloride (3.78 g), 2-naphthaldehyde (10.92 g), 3,4-dimethoxybenzaldehyde (17.43 g), vinylsulfone (7.00 ml) and dimethylformamide (dry DMF, 35 ml) were stirred at room temperature for about 13 days. The reaction mixture was partitioned between chloroform and water and the layers separated. The pooled organic layers were washed with water, dried over anhydrous potassium carbonate and evaporated in vacuo to give about 24.9 g of an oil. The oil was chromatographed to give 1.8 g of crude product which was crystallized from methanol to give 623.2 mg of pure 1-(3,4-dimethoxyphenyl)-4-(2-naphthyl)butan-1,4-dione, m.p. 133° C.

Step B: Preparation of 2-(3,4-dimethylphenyl)-5-(2-naphthyl)tetrahydrofuran

Following the same procedure as described in Example 1, Step B, but substituting for the 1,2-bis-(3,4-dimethoxybenzoyl)ethane used therein, the 1-(3,4-dimethoxyphenyl)-4-(2-naphthyl)butan-1,4-dione prepared above in Step A, there was obtained 370 mg of 2-(3,4-dimethoxyphenyl)-5-(2-naphthyl)tetrahydrofuran as a trans and cis mixture. This mixture was separated by HPLC to the cis-isomer (m.p. 74-77° C) and the transisomer (m.p. 112-113° C).

## EXAMPLE 4

### 2,3-Dimethoxy-5-(tetrahydro-5-(3,4,5-trimethoxyphenyl)-2-furanyl)pyridine

Step A: Preparation of 1-(3,4,5-trimethoxyphenyl)-4-(2,3-dimethoxypyrid-5-yl)-1,4-butanedione

Nine grams of 5,6-dimethoxypyridine-3-carboxaldehyde, 12.6 g 3,4,5-trimethoxyphenyl vinyl ketone, 2.0 g 3-ethyl-5-(2-hydroxyethyl)-4-methylthiazolium bromide, 7.0 g triethylamine in 300 ml of ethanol was refluxed for 24 hours and left at room temperature and upon cooling to room temperature glittering crystals form. The crystals were filtered off and recrystallized from ethanol to yield: 12.5 g, mp. 133-4° C.

Step B: Preparation of 2,3-dimethoxy-5-(tetrahydro-5-(3,4,5-trimethoxyphenyl)-2-furanyl)pyridine

Twelve grams of the above diketone in 200 ml of methanol was treated with 4 x 1 g of sodium borohydride and refluxed for 1 hour. The solvent wa removed and the residue dissolved in ethyl acetate and filtered through a bed of silica. The filtrate, upon evaporation, gave 12.1 g of the diol as viscous liquid. Four grams of this diol, 1.5 g triethylamine, 1.1 g methanesulfonly chloride in 200 ml of methylene chloride was stirred for 30 minutes and then treated with 100 ml of ether. The organic layer was extracted with 3 x 100 ml of 10% sodium hydroxide, dried over sodium sulfate, filtered through a bed of silica and evaporated to yield 2.6 g of an almost colorless residue which was chromatographed on Whatman Magnum-20 column to yield 750 mg of the trans isomer of 2,3-dimethoxy-5-(tetrahydro-5-(2,4,5-trimethoxyphenyl)-2-furanyl)-pyridine and the corresponding cis isomer.

By following a similar sequence of reactions and using 6-methoxypyridine-3-carboxaldehyde instead of 5,6-dimethoxypyridine-3-carboxaldehyde, 2-methoxy-5-(tetrahydro-5-(3,4,5-trimethoxyphenyl)-2-furanyl)-pyridine was prepared.

## EXAMPLE 5

2,5-bis(2,3-dimethoxy-5-pyridyl)tetrahydrofuran(cis and trans isomers)

Step A: Preparation of 1,4-bis(2,3-dimethoxypyrid-5-yl)-1,4-butanedione

One half gram (2,3-dimethoxy-5-pyridyl) vinyl ketone, 0.45 g 5,6-dimethoxypyridine-3-carboxaldehyde, 100 mg 3-ethyl-5-(2-hydroxyethyl)-4-methylthiazolium bromide and 250 mg triethyl amine in 20 ml of absolute ethanol was refluxed for 5 hours and cooled to room temperature. The crystalline diketone was recovered by filtration: mp. 171-3 °C, 306 mg.

Step B: Preparation of trans- and cis-2,5-bis(2,3-dimethoxypyrid-5-yl)tetrahydrofurans

250 mg of the above diketone was reduced with 200 mg of sodium borohydride in methanol as in the previous cases to yield 245 mg of white gummy diol. This diol was dissolved in 20 ml of methylene chloride and treated with 75 mg of triethylamine and 80 mg of methanesulfonyl chloride. After stirring for 30 minutes, solvent was removed and the crude mixture purified by preparative TLC (ethyl acetate-hexane 50/50) to yield 182 mg of the cis trans mixture (Rf 0.48). This mixture was separated by hplc (Whatman Magnum-20 ethyl acetate-hexane 50/50) to yield 38 mg of the trans isomer and 44 mg of the cis isomer.

## EXAMPLE 6

3-(Tetrahydro-5-(3,4,5-trimethoxyphenyl)-2-furan)pyridine

Step A: Preparation of 1-(3,4,5-trimethoxyphenyl)-4-(3-pyridyl)-1,4-butanedione

Three tenth gram sodium cyanide in 15 ml of dimethylformamide (DMF) was added dropwise to 1.3 g of pyridine-3-carboxaldehyde dissolved in 10 ml of DMF. To that, 2.0 g of 3,4,5-trimethoxyphenyl vinyl ketone in 10 ml of DMF added dropwise and the reaction mixture stirred overnight. The next day, 200 ml of distilled water was added and stirring continued while the diketone precipitates out. Filtration and crystallization of the precipitate from methanol gave 1.6 g of the diketone.

Step B: Preparation of trans- and cis-3-(tetrahydro-5-(3,4,5-trimethoxyphenyl)-2-furanyl)pyridine

Following the procedure described in Example 4 Step B, trans- and cis-3-(tetrahydro-5-(3,4,5-trimethoxyphenyl)-2-furanyl)pyridine were prepared.

## EXAMPLE 7

2,5-bis(2-furanyl)tetrahydrofuran

Step A: Preparation of 1,4-bis(2-furanyl)-1,4-butanedione

LDA was prepared from 20 ml of THF, 10.1 g diisopropyl amine and 38.5 ml 2.1 M n-butyl lithium at -10 °C. The temperature was lowered to -40 °C and 11 g of 2-acetyl furan added and stirring continued at

-78°C for 1 hour. Then 13 g of anhydrous cupric chloride was added and stirring continued for 4 hours. To that, 500 ml of 1N HCl was added and the mixture extracted with methylene chloride. The organic layer was dried (sodium sulfate), filtered and evaporated to yield 5.3 g of dark oil. Flash column on a silica column and elution of the Rf .37 band with hexane-ethyl acetate (75/25) gave 830 mg of the desired diketone which was further recrystallized from methylene chloride-hexane: mp. 131-2°C.

By using 12.6 g of 2 acetyl thiophene and following the above procedure, 770 mg of 1,4-bis(2-thienyl)-1,4-butanedione was obtained: mp. 130-1°C.

Step B: Preparation of 2,5-bis(2-furanyl)tetrahydrofuran

Four hundred and forty mg of the above diketone was suspended in 25 ml of THF and reduced to the diol with 20 mg of lithium aluminum hydride at 0°C. The 440 mg of uncrystallized diol obtained as suc was dissolved in 25 ml of methylene chloride and treated with 240 mg triethylamine and 230 mg methanesulfonyl chloride. After an hour, the reaction mixture was treated with 80 ml of ether and the organic layer washed successively with 2 x 50 ml 1N HCl, 2 x 50 ml 5% NaOH, and distilled water. The residue obtained after drying, filtration and evaporation of the organic layer was separated by flash column (silica gel eluted with ethyl acetate-hexane 5/95) to yield 33 mg of the trans isomer and 30 mg of the cis isomer.

Following substantially the same procedures as described in Steps A and B, 160 mg trans and 115 mg cis-tetrahydro-2,5-bis(2-thieno)furan were made from 460 mg of 1,4-bis(2-thienyl)-1,4-butanedione.

EXAMPLE 8

2,6-Dimethoxy-4-(tetrahydro-5-(3,4,5-trimethoxyphenyl)-2-furanyl)pyridine-trans

Step A: Preparation of 2,6-dimethoxyisonicotinaldehyde

2,6-Dimethoxyisonicotinaldehyde was prepared from citrazinic acid according to the known method [E. L. Stogryn, J. Het. Chem., 11, 251 (1974)]: mp. 75-77°C (lit. mp. 74-75°C).

Step B: Preparation of 1-(2,6-dimethoxypyrid-4-yl)-4-(3,4,5-trimethoxyphenyl)-1,4-butanedione

A mixture of 2,6-dimethoxyisonicotinaldehyde (2.11 g), 3,4,5-trimethoxyphenyl vinyl ketone (2.81 g) and 3-ethyl-5-(2-hydroxyethyl)-4-methyl thiazolium bromide (0.42 g) was dissolved in boiling absolute ethanol (15 ml). To this solution was added triethylamine (1.21 g) and the reaction solution was heated to reflux for 14 hours. The reaction mixture was concentrated to dryness and the residue was recrystallized three times from ethanol to give 1.8 g of the diketone mp. 81-83°C.

Step C: Preparation of 1-(2,6-dimethoxypyrid-4-yl)-4-(3,4,5-trimethoxyphenyl)-1,4-butanediol

A solution of 1-(2,6-dimethoxypyrid-4-yl)-4-(3,4,5-trimethoxyphenyl)-1,4-butanedione (1.0 g) in methanol (150 ml) was refluxed with sodium borohydride 0.23 g) for 10 minutes. The mixture was evaporated to dryness. The residue was purified via preparative TLC using 2 mm silica gel plates developed with 5% methanol in methylene chloride to give 1.0 g of the title compound as and oil.

Step D: Preparation of 2,6-dimethoxy-4-(tetrahydro-5-(3,4,5-trimethoxyphenyl)-2-furanyl)pyridine(trans - and cis-)

To a solution of 1-(2,6-dimethoxypyrid-4-yl)-4-(3,4,5-trimethoxyphenyl)-1,4-butanediol (1.0 g) and triethylamine (0.28 g) in methylene chloride (50 ml) at room temperature was added methanesulfonyl chloride (0.28 g) dropwise. The mixture was stirred at room temperature for 30 minutes and quenched with

10% NaOH (10 ml). The organic layer was washed with brine and dried ($Na_2SO_4$). Removal of solvent gave the crude mixture which was purified via preparative TLC using 1 mm silica gel plates developed with 30% ethyl acetate in hexane to give the purified cis-trans product (210 mg) and unreacted starting material (200 mg). The cis-trans mixture was further separated by HPLC using a Partisil 10 column and eluted with 30% ethyl acetate in hexane at 10 ml/minute to give the trans product 4 (80 mg; retention time = 33 minutes) and the cis isomer (100 mg; retention time = 41 minutes).

EXAMPLE 9

2,3-Dimethoxy-6-(tetrahydro-5-(3,4,5-trimethoxyphenyl)-2-furnayl)pyridine-trans

Step A: Preparation of 2,3-dimethoxy-6-pyridinealdehyde

2,3-Dimethoxy-6-pyridinealdehyde was isolated as a by-product from the synthesis of 5,6-dimethoxynicotinaldehyde according to the known method (E. L. Stogryn, J. Het. Chem., 11, 251 (1974)): mp. 86-87° C.

Step B: Preparation of 1-(2,6-dimethoxypyrid-6-yl)-4-(3,4,5-trimethoxyphenyl)-1,4-butanedione

Following essentially the procedure of Example 8, Step B, the title compound was obtained in 70% yield: mp. 130.5-132.5° C.

Step C: Preparation of 1-(2,3-dimethoxypyrid-6-yl)-4-(3,4,5-trimethoxyphenyl)-1,4-butanediol

A solution of the product from Step B (1.25 g) in methanol (50 ml) was refluxed with sodium borohydride (0.26 g) for 10 minutes. The evaporated residue was purified via preparative TLC using 2 mm silica gel plates developed with 60% ethyl acetate in methylene chloride to give 1.1 g of 1-(2,3-dimethoxypyrid-6-yl)-4-(3,4,5-trimethoxyphenyl)-1,4-butanediol as an oil.

Step D: Preparation of 2,3-dimethoxy-6-(tetrahydro-5-(3,4,5-trimethoxyphenyl)-2-furanyl)pyridine-trans

To a solution of the product from last step (0.60 g) and triethylamine (0.22 g) in methylene chloride (30 ml) at room temperature was added methane sulfonyl chloride (0.14 g) during 10 minutes. The mixutre was stirred at room temperature for 1 hour and quenched with 10% NaOH (10 ml). The organic layer was washed with brine and dried ($Na_2SO_4$). Removal of solvent gave the residue which was purified via preparative TLC using 1 mm silica gel plates developed waith 50% ethyl acetate in methylene chloride to give the purified cis-trans product (300 mg) and the recovered starting material 7 (120 mg). The cis-trans mixture was separated by HPLC using a Partisil 10 column and eluted with 35% ethyl acetate in hexane at 10 ml/minute to give the trans product 2,3-dimethoxy-6-(tetrahydro-5-(3,4,5-trimethoxyphenyl)-2-furanyl)-pyridine-trans (140 mg; retention time = 51 minutes) and the cis isomer (135 mg; retention time = 61 minutes).

EXAMPLE 10

2,3,6-Trimethoxy-5-(tetrahydro-5-(3,4,5-trimethoxyphenyl)-2-furanyl)pyridine (trans)

Step A: Prparation of 5,6-dibromo-2,3-dimethoxypyridine

Bromine (10.4 g) in acetic acid (13.5 ml) was added to an acetic acid solution (45 ml), at 10-12° C containing 2,3-dimethoxypyridine (3.4 g) and sodium acetate (5.0 g). The reaction mixture was stirred at room temperature for 3 hours, poured into ice-water and neutralized with 25% sodium hydroxide. Extraction with methylene chloride, evaporation of the solvent and recrystallization of the residue from ether-chloroform gave 1.6 g of 5,6-dibromo-2,3-dimethoxypyridine.

Step B: Preparation of 5-bromo-2,3,6-trimethoxypyridine

A mixture of 5,6-dibromo-2,3-dimethoxypyridine (1.6 g), sodium methoxide (1.5 g) and methanol (2 ml) in dioxane (8 ml) was refluxed for 17 hours. The reaction mixture was concentrated and the residue was extracted with methylene chloride. The extract was filtered through silica gel and evaporated to dryness. The residue was recrystallized from hexane to give 5-bromo-2,3,6-trimethoxypyridine (500 mg): mp. 74.5-75.5° C.

Step C: Preparation of 2,3,6-trimethoxy-5-pyridinealdehyde

A 10% ether solution of 5-bromo-2,3,6-trimethoxypyridine was reacted with a slight excess of n-butyl lithum at -35° C with stirring. The mixture was further stirred at -35° C for 1 hour. A two-fold excess of DMF was added and the reaction allowed to proceed at -20° C for 1 hour. The reaction was treated with an ammonium chloride solution. The solid product was collected, purified to give 2,3,6-trimethoxy-5-pyridinealdehyde.

Step D: Preparation of 1-(2,3,6-trimethoxypyrid-6-yl)-4-(3,4,5-trimethoxyphenyl)-1,4-butanedione

Following substantially the procedure of Example 8, Step B, 1-(2,3,6-trimethoxypyrid-6-yl)-4-(3,4,5-trimethoxyphenyl)-1,4-butanedione was obtained.

Step E: Preparation of 1-(2,3,6-trimethoxypyrid-6-yl)-4-(3,4,5-trimethoxyphenyl)-1,4-butanediol

Following the procedure of Example 9, Step C, the title compound was obtained.

Step F: Preparation of 2,3,6-trimethoxy-5-[tetrahydro-5-(3,4,5-trimethoxyphenyl)-2-furanyl]pyridine-trans

Following substantially the procedure of Example 9, Step D, the trans isomer of the title compound was obtained.

EXAMPLE 11

2-Methoxy-4-[tetrahydro-5-(3,4,5-trimethoxyphenyl)-2-furanyl]thiazole

Step A: Preparation of methyl 2-methoxythiazole-4-carboxylate

Ethyl 2-bromothiazole-4-carboxylate (0.85 g) was refluxed with methoxide methanol solution (0.15 g sodium in 8 ml of methanol) for 30 minutes. The mixture was filtered and the filtrate evaporated to dryness. Purification of the residue via preparative TLC using 2 mm silica gel plates developed with methylene chloride gave pure methyl 2-methoxythiazole-4-carboxylate (0.41 g); m.p. 57-58.5°.

Step B: Preparation of 4-hydroxymethyl-2-methoxythiazole

Methyl 2-methoxythiazole-4-carboxylate (1.7 g) in tetrahydrofuran (50 ml) was treated with lithium aluminum hydride (0.45 g) in portions. After the reaction was complete (ca. 10 min.), the mixture was added saturated sodium sulfate solution dropwise until all the solid turned white. The mixture was filtered and the filtrate evaporated to dryness. The residue was purified via preparative TLC using 2 mm silica gel plates developed with 10% methanol in methylene chloride to give 4-hydroxymethyl-2-methoxythiazole (0.37 g).

Step C: Preparation of 4-formyl-2-methoxythiazole

To a solution of 4-hydroxymethyl-2-methoxythiazole (0.50 g) in pyridine (10 ml) was added lead tetraacetate (1.75 g). The mixture was stirred at 60° for 1-1/2 hours, and quenched with ice-water (50 ml). The mixture was extracted with ether (30 ml x 3). The ether layer was washed with 1N hydrochloric acid (30 ml x 2) and dried ($Na_2SO_4$). Removal of solvent gave the amide product which was purified via preparative TLC using 2 mm silica gel plates developed with methylene chloride to give pure 4-formyl-2-methoxythiazole (0.27 g).

Step D: preparation of 1-(2-methoxythiazol-4-yl)-4-(3,4,5-trimethoxyphenyl)-1,4-butanedione

Following the procedure of Example 8, Step B, the title compound was obtained from 5-formyl-2-methoxythiazole.

Step E: Preparation of 1-(2-methoxythiazol-4-yl)-4-(3,4,5-trimethoxyphenyl)-1,4-butanediol

Following the procedure of Example 8, Step C, but 1-(2-methoxythiazol-4-yl)-4-(3,4,5-trimethoxyphenyl)-1,4-butanediol was obtained from 1-(2-methoxythiazol-4-yl)-4-(3,4,5-trimethoxyphenyl)-1,4-butanedione.

Step F: Preparation of 2-methoxy-4-[tetrahydro-5-(3,4,5-trimethoxyphenyl)-2-furanyl]thiazole-trans

Following the procedure of Example 9, Step D, the trans isomer of the title compound was obtained from 1-(2-methoxythiazol-4-yl)-4-(3,4,5-trimethoxyphenyl)-1,4-butanediol.

## EXAMPLE 12

Trans-2,5-bis(3,4,5-Trimethoxyphenyl)tetrahydrofuran

Step A: Preparation of 3-Dimethylamino-1-(3,4,5-trimethoxyphenyl)-1-propanone

A mixture of 1-(3,4,5-trimethoxyphenyl)-1-ethanone (210 g, 1 mole), dimethylamine hydrochloride, paraformaldehyde (45 g, 1.5 mole) and ethanol (300 ml) containing 1 ml of concentrated hydrochloric acid was heated under reflux in a $N_2$ atmosphere for 1 hour. Paraformaldehyde (30 g, 1 mole) was added, and the heating continued for an additional 2 hours. The warm reaction mixture was poured with vigorous stirring into acetone (2.4 l). The slurry was heated at 60° for 15 minutes, cooled, and filtered. The solid was washed with acetone and dried to give 196 g (65%) of 1, m.p. 175°.

A mixture of 1 (147.5 g, 0.48 moles) in 1N NaOH (750 ml) was shaken with EtOAc (4 x 100 ml). The combined organic extracts were washed with saturated brine, dried ($MgSO_4$) and evaporated in vacuo to give 126 g (97%) of 3-dimethylamino-1-(3,4,5-trimethoxyphenyl)-1-propanone, m.p. 45-47°.

Step B: Preparation of 1,2-bis(3,4,5-Trimethoxybenzoyl)ethane

A solution of 3,4,5-trimethoxybenzaldehyde (16.1 g, 82 mmoles) in DMF (20 ml) was added over 1.5

hours to a mechanically stirred suspension of NaCN (0.4 g, 8 mmoles) in DMF (20 ml) with heating at 35° C in a $N_2$ atmosphere. After an additional 0.5 hour at 35° C, a solution of 3-dimethylamino-1-(3,4,5-trimethoxyphenyl)-1-ethanone(22 g, 82 moles) in DMF (20 ml) was added over 2 hours, and the mixture stirred at 35° C for 18 hours. After dilution with $H_2O$ (400 ml) and acidification with dilute hydrochloric acid, the resulting slurry was filtered, and the solid washed with $H_2O$ and ethanol (4 x 125 ml) and dried to give 26.4 g (77%) of 1,2-bis(3,4,5-trimethoxybenzoyl)ethane, m.p. 193-196° C.

Step C: Preparation of 1,4-bis(3,4,5-trimethoxylphenyl)-1,4-butanediol

1,2-bis(3,4,5-Trimethoxyphenyl)ethane (49.9 g, 0.12 mole) was added in portions over 20 minutes to a slurry of lithium aluminium hydride (9.1 g, 0.24 mole) in THF (625 ml) in $N_2$ atmosphere while the temperature was maintained below 0° C. After one hour at 0° C the mixture was stirred at room temperature for 18 hours. After cooling to 0° C EtOAc (38 ml) was added dropwise, followed by $CH_2Cl_2$ (500 ml) and 5% NaOH (100 ml). The slurry was stirred at room temperature for 30 minutes, filtered, and the solid washed well with $CH_2Cl_2$. The residue after evaporation of the filtrate in vacuo was dissolved in $CH_2Cl_2$, washed with $H_2O$, dried ($MgSO_4$) and evaporated in vacuo. Crystallization of the residue from hexane-$CH_2Cl_2$ gave 40.1 g (80%) of 1,4-bis(3,4,5-trimethoxyphenyl)-1,4-butanediol, m.p. 127-130°.

Step D: Preparation of trans-2,5-bis(3,4,5-trimethoxyphenyl)tetrahydrofuran

To a solution of 1,4-bis(3,4,5-trimethoxyphenyl)-1,4-butanediol (103 g, 0.24 moles) in $CHCl_3$ (1030 ml) cooled to -30° C was added over 0.5 hour a 10% (v/v) solution of trifluoroacetic acid in $CHCl_3$ (1030 ml). The mixture was kept at -20° for 90 hours and then washed with ice-cold 5% NaOH (2100 ml), $H_2O$ (200 ml), dried ($MgSO_4$) and evaporated in vacuo. The syrupy residue (108 g) was chromatographed on silica gel (1100 g). After elution first with $CH_2Cl_2$ (2 l) and 1:3 EtOAc-hexane (4 l), the crude product (81 g) was eluted with 1:1 EtOAc-hexane. Trituration with hot hexane (3 x 750 ml) gave 63.8 g of crude crystalline product. Three recrystallizations from cyclohexane-EtOAc (20:1) gave pure trans-2,5-bis(3,4,5-trimethoxyphenyl)tetrahydrofuran containing less than 1% cis isomer, m.p. 140-142° C. Calc. for $C_{22}H_{28}O_7$: C, 65.33; H, 6.98.
Found: C, 65.60; H, 6.80

Following substantially the same procedure as described in Steps A-D, there were prepared the following related compounds:

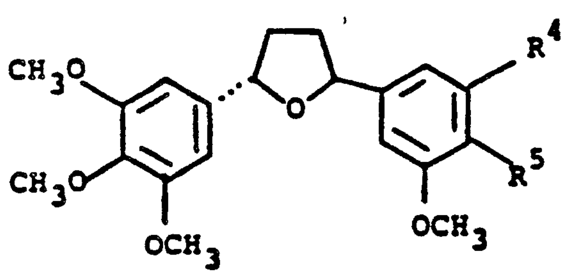

| $R^4$ | $R^5$ | Rabbit platelet membrane inhibition $IC_{50}$ (nM) | Physical Properties |
|---|---|---|---|
| I | $OCH_2CH=CH_2$ | 13.0 | m.p. 85-86°C |
| $OCH_3$ | $OCH_2$– (cyclopropyl) | 8.3 | oil |
| $OCH_3$ | $OCH_2CH=CH_2$ | 58.0 | oil |

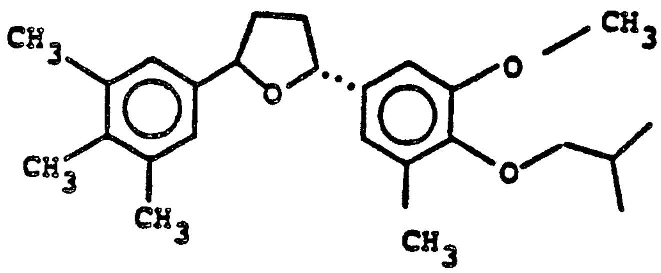

NMR ($CDCl_3$):
δ0.22 - 0.30 (m, 2H, cyclopropane C-1 + C-2 (CH's(9))
0.48 - 0.58 (m, 2H, cyclopropane C-1 + C-2 CH's (6))
1.20 - 1.32 (m, 1H, cyclopropane C-3)
1.86 - 2.10 (m, 2H, THF C-3 and C-4 (c+d))
2.32 - 2.56 (m, 2H, THF C-3 and C-4 (c+d))
3.8 (d, 2H, cyclopropyl methyl $CH_2$'s)
3.88 (s, 6H, e & f methoxy $CH_3$'s)
3.90 (s, 6H, 3 & f methoxy $CH_3$'s)
3.86 (s, 3H, g methoxy $CH_3$'s)
5.16 - 5.26 (m, 2H, THF C-2 + C-5 CH's)
6.64 (s, 2H aromatic protons)
6.66 (s, 2H aromatic protons)

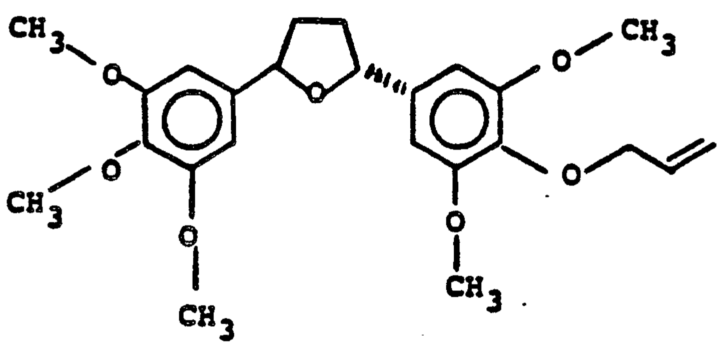

NMR ($CDCl_3$):
δ1.9 - 2.12 (m, 2H, THF C-3 + C-4 (d + e))

2.4 - 2.56 (m, 2H, THF C-3 + C-4 (d + e))
3.86 (s, 3H, h methoxy $CH_3$'s)
3.88 (s, 6H, methoxy $CH_3$'s (g + f))
3.90 (s, 6H, methoxy $CH_3$'s (g + f))
4.52 (d, 2H, C-1 of allyloxy group)
5.18 - 5.28 (m, 2H, C-2 + C-5 THF CH's)
5.14 - 5.38 (m, 2H, C-3 $CH_2$'s of allyloxy group (a + b))
6.04 - 6.22 (m, 1H, C-2 CH of allyloxy group)
6.66 (s, 4H, aromatic protons)

## EXAMPLE 13

### Step A: Preparation of 4-Allyloxy-5-iodo-3-methoxybenzaldehyde

Allyl bromide (27.5 ml, 0.32 mol) was added to a solution of 5-iodovanillin (50.0 g, 0.18 mol) in DMF (200 ml) containing potassium carbonate (48 g, 0.35 mol) at 80°C. The mixture was stirred at this temperature for 1 hour, filtered, and the filtrate was evaporated to a residue, which was purified by HPLC (hexane-ethyl acetate, 4:1 v/v) to give 4-allyloxy-5-iodo-3-methoxybenzaldehyde (48 g, 84%).

### Step B: Preparation of 1-(4-Allyloxy-5-iodo-3-methoxyphenyl)-4-(3,4,5-trimethoxyphenyl)-1,4-butanedione

A mixture of 4-allyloxy-5-iodo-3-methoxybenzaldehyde (46 g, 0.15 mol), 3,4,5-trimethoxyphenyl vinyl ketone (38 g, 0.16 mol) and thiazole catalyst (6 g, 0.07 mol) in triethylamine (200 ml) was heated, with stirring, at 70°C for 2 hours, and kept at room temperature overnight. Ethanol was added to the solid mass, filtered, and the filtrate was evaporated to a residue, which was purified by HPLC (hexane-ethyl acetate; 2:1, v/v). The combined yield of 1-(4-allyloxy-5-iodo-3-methoxyphenyl)-4-(3,4,5-trimethoxyphenyl)-1,4-butanedione was 65 g (83%), m.p. 113-114°C.

### Step C: Preparation of 1-(4-Allyloxy-3-methoxy-5-methylthiophenyl)-4-(3,4,5-trimethoxyphenyl)-1,4-butanedione

A mixture of 1-(4-allyloxy-5-iodo-3-methoxyphenyl)-4-(3,4,5-trimethoxyphenyl)-1,4-butanedione (5.0 g, 9.3 mmol) and copper thiomethyl (3.0 g, 27.1 mmol) in N-methyl-2-pyrrolidinone (50 ml) was heated at 158°C (bath temperature) for 2 hours, cooled, and evaporated to dryness. The reaction mixture was separated by Preparative HPLC (dichloromethane-ethyl acetate; 98:2, v/v) to give the unreacted starting material (1.5 g), 1-(4-allyloxy-3-methoxy-5-methylthiophenyl)-4-(3,4,5-trimethoxyphenyl)-1,4-butanedione (1.2 g, 40% based on unreacted starting material); n.m.r ($CDCl_3$): δ2.49 (s, $SCH_3$), 3.45 (s, $CH_2CH_2$), 3.93, 3.94, 3.96 (3s, 4 $OCH_3$), 4.65 (d, $\underline{CH_2}CH=CH_2$), 5.21-5.45 ($CH_2CH=\underline{CH_2}$), 6.13 (m, $CH_2\underline{CH}=CH_2$), 7.27 (s, ArH-$C_4$), 7.41, 7.49 (2d, ArH-$C_1$).

### Step D: Preparation of 1-(4-Allyloxy-3-methoxy-5-methylthiophenyl)-4-(3,4,5-trimethoxyphenyl)-1,4-butanediol

Lithium aluminum hydride (100 mg) was added to a solution of 1-(4-allyloxy-3-methoxy-5-methylthiophenyl)-4-(3,4,5-trimethoxyphenyl)-1,4-butanedione (1.0 g, 2.2 mmol) in tetrahydrofuran (10 ml) at 0°C. The mixture was stirred at room temperature for 0.5 hour and quenched with 2N sodium hydroxide. The cake was filtered and washed with THF-$Et_2O$ (1:1, v/v). The combined filtrates were evaporated to give 1-(4-allyloxy-3-methoxy-5-methylthiophenyl)-4-(3,4,5-trimethoxyphenyl)-1,4-butanediol (1.0 g, 99%), which was used directly in the next experiment without further purification. The title compound had n.m.r. ($CDCl_3$): δ0.88 (b, $CH_2CH_2$), 2.43 (s, $SCH_3$), 3.85, 3.86, 3.87 (3s, 4 $OCH_3$), 4.54 (d, $\underline{CH_2}CH=CH_2$), 4.72 (b, 2 CHOH), 5.22-5.45 ($CH_2CH=\underline{CH_2}$), 6.16 (m, $CH_2\underline{CH}-CH_2$), 6.60, 6.74 (2s, ArH).

Step E: Preparation of Cis- and trans-2-(4-allyloxy-3-methoxy-5-methylthiophenyl)-5-(3,4,5-trimethoxyphenyl)tetrahydrofurans

Trifluroacetic acid (10% in chloroform, 10 ml) was added to a solution of 1-(4-allyloxy-3-methoxy-5-methylthiophenyl)-4-(3,4,5-trimethoxyphenyl)-1,4-butanediol (1.0 g, 2.2 mmol) in chloroform (15 ml) at 0 °C. The solution was kept at room temperature for 2 hours, diluted with chloroform and washed with diluted sodium hydroxide and water. The organic layer was dried and evaporated to a residue, which was put on a flash column of silica gel and eluted with hexane-ethyl acetate (2:1, v/v) to give the trans isomer, (417 mg, 43%; a more mobile component) and the cis isomer, (208 mg, 22%). Cis-2-(4-allyloxy-3-methoxy-5-methylthiophenyl)-5-(3,4,5-trimethoxyphenyl)-tetrahydrofuran had n.m.r. ($CDCl_3$): δ2.02, 2.45 (2m, $CH_2CH_2$), 2.40 (s, $SCH_3$), 3.86, 3.87 (2s, 4 $OCH_3$), 4.55 (br d, $CH_2CH=CH_2$), 5.07 (H-2, H-5, br t), 5.23-5.46 ($CH_2CH=CH_2$), 6.17 (m, $CH_2CH=CH_2$), 6.69 (s, ArH-$C_5$), 6.83-6.85 (2d, ArH-$C_2$). The corresponding trans isomer had n.m.r ($CDCl_3$); δ2.01, 2.49 (2m, $CH_2CH_2$), 2.46 (s, $SCH_3$), 3.87, 3.90, 3.91 (3s, 4 $OCH_3$), 4.56 (br d, $CH_2CH=CH_2$), 5.23 (H-2, H-5, br t), 5.23-5.46 ($CH_2CH=CH_2$), 6.18 (m, $CH_2CH=CH_2$), 6.67 (s, ArH-$C_5$), 6.82-6.83 (2d, ArH-$C_2$).

## EXAMPLE 14

Trans-2-(4-allyloxy-3-methoxy-5-methylsulfinylphenyl)-5-(3,4,5-trimethoxyphenyl)tetrahydrofuran

3-Chloroperbenzoic acid (33 mg, 0.19 mmol) was added to stirred solution of trans-2-(4-allyloxy-3-methoxy-5-methylthiophenyl-5-(3,4,5-trimethoxyphenyl)tetrahydrofuran (80 mg, 0.18 mmol) in dichloromethane (3 ml). After 0.5 hour, the solution was diluted with dichloromethane and washed with aqueous sodium hydrogencarbonate, water, dried and evaporated to a residue. The product was purified by flash column chromatography (hexane-ethyl acetate; 1:1, v/v) to give trans-2-(4-allyloxy-3-methoxy-5-methylsulfinylphenyl)-5-(3,4,5-trimethoxyphenyl)tetrahydrofuran (56 mg, 68%) as an isomeric mixture.

## EXAMPLE 15

Trans-2-(4-allyloxy-3-methoxy-5-methylsulfonylphenyl)-5-(3,4,5-trimethoxyphenyl)tetrahydrofuran

3-Chloroperbenzoic acid (62 mg, 0.36 mmol) was added to a stirred solution of trans-2-(4-allyloxy-3-methoxy-5-methylthiophenyl-5-(3,4,5-trimethoxyphenyl)tetrahydrofuran (80 mg, 0.18 mmol) in dichloromethane (4 ml). After 1 hour, the reaction mixture was worked up as usual and purified by flash column chromatography (hexane-ethyl acetate; 2:1, v/v) to give trans-2-(4-allyloxy-3-methoxy-5-methylsulfonylphenyl)-5-(3,4,5-trimethoxyphenyl)tetrahydrofuran (60 mg, 70%), which crystallized upon standing, m.p. 97-98 °C ($Et_2O$-Pet.$Et_2O$); n.m.r. ($CDCl_3$): δ1.99, 2.49 (2m, $CH_2CH_2$), 3.25 (s, $SO_2CH_3$), 3.84, 3.88, 3.93 (3s, 4 $OCH_3$), 4.67 (2t, J 6.0, 1.0 Hz $CH_2CH=CH_2$), 5.15-5.26 (H-2, H-5, $CH_2CH=CH_2$), 6.18 (m, $CH_2CH=CH_2$), 6.61 (s, ArH-$C_5$), 7.27, 7.51 (2d, J 2.0 Hz, ArH-$C_2$).

## EXAMPLE 16

Trans-2-(3-methoxy-5-methylthio-4-propoxyphenyl)-5-(3,4,5-trimethoxyphenyl)tetrahydrofuran

A solution of trans-2(4-allyloxy-3-methoxy-5-methylthiophenyl-5-3,4,5-trimethoxyphenyl)-tetrahydrofuran (100 mg) in ethyl acetate (3 ml) containing 10% palladium-on-charcoal (80 mg) was hydrogenated for 1 hour. The mixture was filtered and washed with ethyl acetate. The combined filtrates were evaporated to give trans-2-(3-methoxy-5-methylthio-4-propoxyphenyl)-5-(3,4,5-trimethoxyphenyl)-tetrahydrofuran (90 mg, 90%), which was used directly in the next example without further purification.

## EXAMPLE 17

Trans-2-(3-methoxy-5-methylsulfonyl-4-propoxypheyl)-5-(3,4,5-trimethoxyphenyl)tetrahydrofuran

3-Chloroperbenzoic acid (44 mg, 0.25 mmol was added to a stirred solution of the product from Example 16 (54 mg, 0.12 mmol) in dichloromethane (4 ml). After 1 hour, the reaction mixture was worked up as usual and purified by flash column chromatography (hexane-ethyl acetate; 3:1, v/v) to give trans-2-(3-methoxy-5-methylsulfonyl-5-propoxyphenyl)-5-(3,4,5-trimethoxyphenyl)tetrahydrofuran (40 mg, 69%), which crystallized upon standing, m.p. 95-96 °C ($Et_2O$-Pet.$Et_2O$); n.m.r. ($CDCl_3$): δ1.05 (t, J 7.5 Hz, $CH_3$), 1.89 (m, $CH_2CH_2CH_3$), 1.89, 2.49 (2m, H-3, H-4), 3.26 (s, $SO_2CH_3$), 3.85, 3.89, 3.93 (3s, 4 $OCH_3$), 4.12 (t, $CH_2\overline{CH_2}CH_3$), 5.16-5.28 (H-2, H-5), 6.62 (s, ArH-$C_5$), 7.27, 7.51 (2d, ArH-$C_2$).

## EXAMPLE 18

Alternative Synthesis of Trans-2-(3-methoxy-5-methylthio-4-propoxyphenyl)-5-(3,4,5-trimethoxyphenyl)-tetrahydrofuran

Step A: Preparation of 1-(5-iodo-3-methoxy-4-propoxyphenyl)-4-(3,4,5-trimethoxyphenyl)-1,4-butanedione

A solution of 5-iodo-3-methoxy-4-propoxybenzaldehyde (26 g, 0.08 mol) in DMF (75 ml) was added over 15 minutes to a stirred solution of sodium cyanide (4 g, 0.08 mol) in DMF (100 ml) at 35 °C. After stirring for 20 minutes, a solution of 3-dimethylamino-1-(3,4,5-trimethoxyphenyl)-1-propanone (18 g, 0.07 mol) in DMF (100 ml) was added over 0.5 hour. The mixture was stirred at 35 °C for 1 hour and kept at room temperature overnight. It was poured into ice-cold 20% hydrochloric acid (2 1), and the solid was filtered, dried by suction, and taken up in ethyl acetate. The organic layer was dried over magnesium sulfate, filtered, and the filtrate was evaporated to dryness. Crystallization from $Et_2O$-hexane gave 1-(5-iodo-3-methoxy-4-propoxyphenyl)-4-(3,4,5-trimethoxyphenyl)-1,4-butanedione (24 g, 61%), m.p. 120-121 °C.

Step B: Preparation of 1-(3-methoxy-5-methylthio-4-propoxyphenyl)-4-(3,4,5-trimethoxyphenyl)-1,4-butanedione

A mixture of copper (10 g, 0.16 mol), methyl disulfide (10 ml, 0.11 mol) in 2,4-lutidine (100 ml) was heated with stirring at 125 °C for 2 hours. 1-(5-Iodo-3-methoxy-4-propoxyphenyl)-4-(3,4,5-trimethoxyphenyl)-1,4-butanedione (14 g, 0.03 mol) was added to the mixture, and the contents were heated at 160 °C for 16 hours. The reaction mixture was filtered, and the filtrate was concentrated to a residue, which was taken up in dichloromethane. The organic phase was filtered through silica gel (100 g), and the solid was washed with dichloromethane and ethyl acetate. The combined filtrates were evaporated to dryness. Ethyl ether was added and crystals were collected (8.85 g, 73%), m.p. 113-114 °C.

Step C: Preparation of 1-(3-methoxy-5-methylthio-4-propoxyphenyl)-4-(3,4,5-trimethoxyphenyl)-1,4-butanediol

The title compound was prepared following the same procedure as described in Example 13, Step D in near quantitative yield.

Step D: Preparation of Cis- and trans-2-(3-methoxy-5-methylthio-4-propoxyphenyl)-5-(3,4,5-trimethoxyphenyl)tetrahydrofuran

The title compounds were prepared similarly by the procedures described in Example 13, Step E in

65% yield (trans:cis, 2:1).

## EXAMPLE 19

### Trans-2-(4-Allyloxy-3-methoxy-5-nitrophenyl)-5-(3,4,5-trimethoxyphenyl)tetrahydrofuran

#### Step A: Preparation of 4-allyloxy-3-methoxy-5-nitrobenzaldehyde

5-Nitrovanillin was treated with excess allyl bromide in dry DMF in the presence of potassium carbonate to give 4-allyloxy-3-methoxy-5-nitrobenzaldehyde: mp 67-68° Anal. Calcd for $C_{11}H_{11}NO_5$: C, 55.69; H, 4.67; N, 5.91.
Found: C, 55.56; H, 4.64; N, 5.76.

#### Step B: Preparation of 1-(4-allyloxy-3-methoxy-5-nitrophenyl)-4-(3,4,5-trimethoxyphenyl)-1,4-butanedione

A mixture of trimethoxyphenyl vinyl ketone (2.22 g, 10 mmol), the aldehyde of Step A (2.37 g, 10 mmole) and thiazolium bromide catalyst (0.30 g) was mixed thoroughly at room temperature for 5-10 minutes to a viscous mass. To this mixture was added triethylamine (1.50 ml) and again mixed thoroughly at room temperature for 10 minutes. The mixture was then heated at 70-80° for 5 hours. The resulting reaction mixture was triturated with absolute ethanol (10 ml) in the cold. The solid product was collected and recrystallized from methanol to give 2.3 g of pure 1-(4-allyloxy-3-methoxy-5-nitrophenyl)-4-(3,4,5-trimethoxyphenyl)-1,4-butanedione (50% yield): mp 142.5-143.5°. Anal. Calcd for $C_{23}H_{25}NO_9$: C, 60.12; H, 5.48; N, 3.05.
Found: C, 60.16; H, 5.46; N, 2.79.

#### Step C: Preparation of 1-(4-allyloxy-3-methoxy-5-nitrophenyl)-4-(3,4,5-trimethoxyphenyl)-1,4-butanediol

The diketone 1-(4-allyloxy-3-methoxy-5-nitrophenyl)-4-(3,4,5-trimethoxyphenyl)-1,4-butanedione (1.6 g) in tetrahydrofuran (100 ml) at 0 to 5° was treated with $LiAlH_4$ (0.2 g) at 0 to 5° for $\frac{1}{2}$ hour and at room temperature for 1 hour. After the work up, the crude mixture was purified via preparative tlc using silica gel plates (2000 µm) developed with 20% hexane in ethyl acetate. The diol 1-(4-allyloxy-3-methoxy-5-nitrophenyl)-4-(3,4,5-trimethoxyphenyl)-1,4-butanediol (1.4 g) was obtained 87% yield: mp. 110-111.5°. Anal Calcd for $C_{23}H_{29}NO_9$: C, 59.60; H, 6.31; N, 3.02.
Found: C, 59.45; H, 6.33; N, 3.00.

#### Step D: Preparation of Trans-2-(4-allyloxy-3-methoxy-5-nitrophenyl)-5-(3,4,5-trimethoxyphenyl)-tetrahydrofuran

The diol 1-(4-allyloxy-3-methoxy-5-nitrophenyl)-4-(3,4,5-trimethoxyphenyl)-1,4-butanediol (0.71 g) in methylene chloride (50 ml) was added 10% trifluoroacetic acid (6 ml) at room temperature. The mixture was stirred at room temperature for 1-3 hours until the complete disappearance of the product of Example 19, Step C and then quenched with cold 1N NaOH (15 ml). The organic layer was separated, dried ($Na_2SO_4$). Removal of solvent gave the crude cis-transmixture which was carefully recrystallized from hexane-methylene chloride to give the fine needles of trans-2-(4-allyloxy-3-methoxy-5-nitrophenyl)-5-(3,4,5-trimethoxy-phenyl)tetrahydrofuran (0.30 g); mp 119-120°.

## EXAMPLE 20

Trans-2-(4-Alkoxy-3-methoxy-5-nitrophenyl)-5-(3,4,5-trimethoxyphenyl)tetrahydrofuran

Step A: Preparation of trans-2-(4-hydroxy-3-methoxy-5-nitrophenyl)-5-(3,4,5-trimethoxyphenyl)-tetrahydrofuran

Trans-2-(4-Allyloxy-3-methoxy-5-nitrophenyl)-5-(3,4,5-trimethoxyphenyl)tetrahydrofuran (1.1 g) in t-butanol (40 ml) was treated with excess potassium t-butoxide under nitrogen in a sealed tube. The mixture was heated at 105° for 2 hours and cooled to room temperature. The reaction mixture was poured onto ice-water, and acidified with acetic acid. The resulting mixture was extracted with methylene chloride and dried over anhydrous sodium sulfate. Removal of solvent gave the crude product which was purified via preparative tlc using silica gel plates (2000 µm) developed with 30% hexane in ethyl acetate. Trans-2-(4-hydroxy-3-methoxy-5-nitrophenyl)-5-(3,4,5-trimethoxyphenyl)tetrahydrofuran (0.92 g) was isolated in ca. 95% purity which was used for the preparation of a series of 4-alkoxy-3-methoxy-5-nitrophenyl analogs. Recrystallization from methanol gave trans-2-(4-hydroxy-3-methoxy-5-nitrophenyl)-5-(3,4,5-trimethoxyphenyl)tetrahydrofuran: mp about 260° (dec).

Step B: Preparation of Trans-2-(4-Alkoxy-3-methoxy-5-nitrophenyl)-5-(3,4,5-trimethoxyphenyl)tetrahydrofuran

Trans-2-(4-Hydroxy-3-methoxy-5-nitrophenyl)-5-(3,4,5-trimethoxyphenyl)tetrahydrofuran (50 mg), alkyl iodide or bromide (0.5 ml) and pulverized potassium carbonate (1.0 g) in dry DMF (1.5 ml) were stirred at room temperature for 16-6 hours. The mixture was quenched in water, and extracted with methylene chloride. The isolated product was further purified via preparative tlc using silica gel plates (1000 µm) developed with 30% hexane ethyl acetate to give trans-2-(4-alkoxy-3-methoxy-5-nitrophenyl)-5-(3,4,5-trimethoxyphenyl)tetrahydro-furan.

The following are a few selected examples:

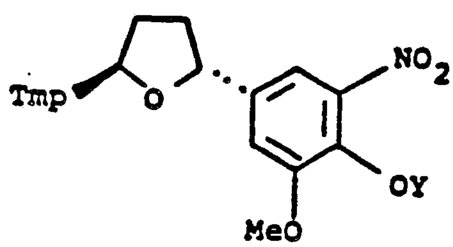

(Tmp = 3,4,5-trimethoxyphenyl)

| Y | mp |
|---|---|
| $CH_3$ | 83.5-85.5° |
| $CH_2CH_2CH_3$ | 112.5-114.5° |
| $CH_2CH_2CH_2CH_3$ | 112-114° |
| $CH_2CH_2CH_2CH_2CH_3$ | 108.5-110.5 |
| $CH_2COOCH_2CH_3$ | 87-88.5° |
| 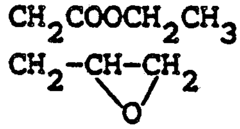 | 125.5-127° |

## EXAMPLE 21

Trans-2-(4-Allyloxy-5-N-alkylamino-3-methoxyphenyl)-5-(3,4,5-trimethoxyphenyl)tetrahydrofuran

Step A: Preparation of Trans-2-(4-allyloxy-5-amino-3-methoxyphenyl)-5-(3,4,5-trimethoxyphenyl)-tetrahydrofuran

Trans-2-(4-Allyloxy-3-methoxy-5-nitrophenyl)-5-(3,4,5-trimethoxyphenyl)tetrahydrofuran (1.0 g) in THF (50 ml) and glacial AcOH (16 ml) at 10-15° was added powdered zinc (3.5 g) in portions over 10-15 minutes (slight exothermic). The mixture was stirred at ambient temperature (25-35°) for 2 hours and filtered. The filtrate was concentrated *in vacuo* to dryness. The residue was taken up in methylene chloride and filtered. The organic solution was concentrated to dryness and the residue recrystallized from hexane-methylene chloride to give 0.86 g of trans-2-(4-allyloxy-5-N-alkylamino-3-methoxyphenyl)-5-(3,4,5-trimethoxyphenyl)tetrahydrofuran (92% yield): mp 94-95°.

Step B: Preparation of Trans-2-(4-Allyloxy-5-ethoxycarbonylmethylamino-3-methoxyphenyl)-5-(3,4,5-trimethoxyphenyl)tetrahydrofuran

Trans-2-(4-Allyloxy-5-amino-3-methoxyphenyl)-5-(3,4,5-trimethoxyphenyl)tetrahydrofuran (50 mg), ethyl bromoacetate (0.7 ml) and pulverized potassium carbonate (1. 0 g) in dry DMF (2 ml) were stirred at room temperature for 20 hours. The mixture was evaporated in high vacuum and the residue purified *via* preparative tlc using a silica gel plate (1000 µm) developed with 30% ethyl acetate in hexane to give trans-2-(4-allyloxy-5-ethoxycarbonylmethyl amino-3-methoxyphenyl)-5-(3,4,5-trimethoxyphenyl)-tetrahydrofuran (30 mg) as an oil: mass m/e 501.

Following essentially the same procedures, the following compounds were prepared:

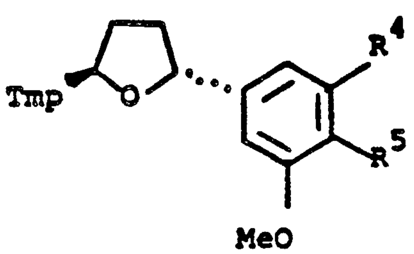

| $R^4$ | $R^5$ | mp |
|---|---|---|
| $NH_2$ | $OCH_2CH_2CH_3$ | 98.5-99.5° |
| $N(CH_3)_2$ | $OCH_2CH=CH_2$ | mass m/e 459 |
| $\oplus N(CH_3)_3 I^{\ominus}$ | $OCH_2CH=CH_2$ | 110-112° |
| $NMe-SO_2Me$ | $OCH_2CH=CH_2$ | 103-104° |

## EXAMPLE 22

Trans-2-(4-Allyloxy-5-methanesulfonylamino-3-methoxyphenyl)-5-(3,4,5-trimethoxyphenyl)tetrahydrofuran

To a stirred solution of trans-2-(4-allyloxy-5-amino-3-methoxyphenyl)-5-(3,4,5-trimethoxyphenyl)-tetrahydrofuran (43 mg) in methylene chloride (2 ml) containing triethylamine (36 mg) at 0 to 5° was added a solution of methanesulfonyl chloride (20 mg) in methylene chloride (0.5 ml) dropwise. The mixture was stirred at 5° for 1 hour at ca. 70% conversion. The reaction solution was quenched with 1% NaOH solution (0.3 ml). The organic solution was dried, concentrated and the residue purified via preparative tlc using silica gel plates (1000 µm) developed with 50% ethyl acetate in hexane to give trans-2-(4-allyloxy-5-methanesulfonylamino-3-methoxyphenyl)-5-(3,4,5-trimethoxyphenyl)tetrahydrofuran (23 mg): mp 149-150.5°.

Following essentially the same procedures, the following compounds were prepared:

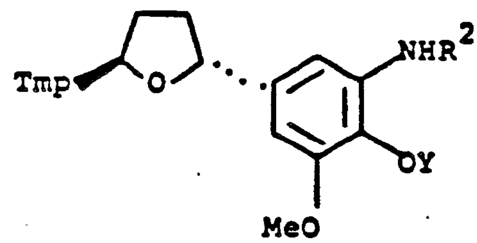

| Y | $R^2$ | mp |
|---|---|---|
| $COCH_3$ | $CH_2CH_2CH_3$ | m/e 459 |
| $SO_2CH_3$ | $CH_2CH_2CH_3$ | 115.5-117.5° |

EXAMPLE 23

Trans-2-(3,4,5-timethoxyphenyl)-5-(3-methoxy-4-propoxy-5-carboxamidophenyl)-tetrahydrofuran

Step A: Preparation of 1-(3,4,5-trimethoxyphenyl)-4-(3-methoxy-4-propoxy-5-cyanophenyl)-1,4-butanedione

A mixture of 1-(3,4,5-trimethoxyphenyl)-4-(3-methoxy-4-propoxy-5-cyanophenyl)-1,4-butanedione (20.0 g, 36 mmole) and CuCN (30.0 g, 330 mmole) was suspended in 200 ml dry N-methylpyrolidinone, heated to 140° C under nitrogen atmosphere for two hours. The dark reddish brown solution was cooled and poured into 500 ml of ice/water. Extracted twice with 500 ml portions of $CH_2Cl_2$. The combined extracts were filtered through celite and then washed three times with 100 ml portions of saturated NaCl solutions. The methylene chloride solution was then filtered through a plug of silica (200 g) to remove red color. The filtrate was concentrated to a light orange color oil. It was crystallized from ethyl acetate/hexane to yield 13.5 g of 1-(3,4,5-trimethoxyphenyl)-4-(3-methoxy-4-propoxy-5-cyanophenyl)-1,4-butanedione (83% yield).

Step B: Preparation of 1-(3,4,5-trimethoxyphenyl)-4-(3-methoxy-4-propoxy-5-cyanophenyl)-1,4-butanediol

A mixture of 1-(3,4,5-trimethoxyphenyl)-4-(3-methoxy-4-propoxy-5-cyanophenyl)-1,4-butanedione (13.5 g, 30 mmole) and $NaBH_4$ (2.0 g, 53 mmole) in 100 ml of absolute ethanol and 25 ml ethyl acetate was stirred under nitrogen atmosphere. The temperature was raised to 40° C and maintained for one hour. The cooled reaction mixture was added to 200 ml icy water and was extracted twice with 300 ml portions of methylene chloride. The combined methylene chloride extracts were dried with anhydrous sodium sulfate

and concentrated to a yellow oil (12.0 g, 89% yield). The product was used directly in the next reaction without further purification.

Step C: Preparation of Trans-2-(3,4,5-trimethoxyphenyl)-5-(3-methoxy-4-propoxy-5-cyanophenyl)-tetrahydrofuran

In a round bottom flask, 1-(3,4,5-trimethoxyphenyl)-4-(3-methoxy-4-propoxy-5-cyanophenyl)-1,4-butanediol (12.0 g, 27 mmole) dissolved in 75 ml chloroform was stirred at room temperature under nitrogen atmosphere. A 10% solution of trifluoroacetic acid in chloroform (75 ml) was added slowly. The mixture stirred for three hours. Twenty milliliters more of 10% TFA solution added and stirred for additional 30 minutes. A solution of 10% NaOH in water (200 ml) added. The cholorform layer concentrated to a colorless oil. Flash column chromatography (30% EtOAc/hexane) separated trans- product from cis- product (2:1 ratio) and six grams of desired trans- product trans-2-(3,4,5-trimethoxyphenyl)-5-(3-methoxy-4-propoxy-5-cyanophenyl)tetrahydrofuran was recovered after recrystallization from $CH_2Cl_2$/hexane. (53% yield) m.p. 128-130 °C. Cis-isomer was recovered as a by-product (28% yield).

Step D: Preparation of Trans-2-(3,4,5-trimethoxyphenyl)-5-(3-methoxy-4-propoxy-5-carboxyamidephenyl)-tetrahydrofuran

A suspension of trans-2-(3,4,5-trimethoxyphenyl)-5-(3-methoxy-4-propoxy-5-cyanophenyl)-tetrahydrofuran (5.1 grams, 14 mmole) in 100 ml ethanol and 25 ml 20% NaOH in water was heated to reflux for two hours. An additional 10 ml 20% NaOH in water added and refluxed for another one and half hours. Cooled reaction mixture was concentrated and extracted 3 times with 100 ml portions of $CH_2Cl_2$. Flash column chromatography (50% EtOAc/hexane) yielded 3,2 grams of trans-2-(3,4,5-trimethoxyphenyl)-5-(3-methoxy-4-propoxy-5-carboxyamide-phenyl)-tetrahydrofuran (60% yield) after recrystallization from EtOAc/hexane. m.p. 108-110 °C.

EXAMPLE 24

Trans-2-(5-(2-hydroxyethylsulfonyl)-4-propoxy-3-methoxyphenyl)-5-(3,4,5-trimethoxyphenyl)-tetrahydrofuran

Step A: Preparation of 1-(5-iodo-4-propoxy-3-methoxyphenyl)-4-(3,4,5-trimethoxyphenyl)-butan-1-4-dione

n-Propyl bromide (29.2 mL, 0.32 mole) was added to a solution of 1-(3-iodo-4-hydroxy-5-methoxyphenyl)-4-(3,4,5-trimethoxyphenyl)butan-1,4-dione (100 g, 0.2 mole) in DMF (400 mL) containing potassium carbonate (96 g, 0.35 mole). The mixture was stirred at 70 °C for 2 hours during which time starting material disappeared. The mixture was cooled, filtered, and extracted with ethyl acetate, and the extract was washed with water and brine, dried over anhydrous $MgSO_4$, and evaporated in vacuo to afford the crude product. Crystallization from ether/hexane gave a near quantitative yield of the title compound as white crystals, m.p. 120-121 °C. NMR ($CDCl_3$): $\delta$ 1.08 (t, J=7.5Hz, $CH_2CH_2CH_3$) 1.87 (m, $CH_2CH_2CH_3$), 2.41 (t, J=4Hz, $COCH_2CH_2CO$), 3.90 and 3.97 (s, u Ar $OCH_3$), 4.04 (t, J=8Hz, $OCH_2CH_2CH_3$), 7.28 (s, trimethoxyphenyl H-2, H-6), 7.51 (d, J=1.5Hz, Iodophenyl 'H-6), 8.06 (d, J=1.5Hz, Iodophenyl 'H-2).

Step B: Preparation of 1-(5-(2-hydroxyethylthio-4-Propoxy-3-methoxyphenyl)-4-(3,4,5-trimethoxyphenyl)-butan-1,4-dione

1-(5-Iodo-4-propoxy-3-methoxyphenyl)-4-(3,4,5-trimethoxyphenyl)butan-1,4-dione (108 g, 0.2 mole) was dissolved in DMF (800 mL), metallic copper powder (108 g) was added, and the mixture was stirred vigorously at 130-140 °C under nitrogen atmosphere. A solution of 2-hydroxyethyldisulfide (50 g, 0.32 mole) in DMF (200 mL) was added and the resulting mixture was stirred vigorously at 130-140 °C overnight. The reddish-brown reaction mixture was filtered and the filtrate was extracted with ethyl acetate, washed with

$H_2O$ (5 x 1 L) and brine, dried over anhydrous $MgSO_4$, and evaporated in vacuo to provide a dark brown oil. The oil was crystallized from ether/hexane to afford a pale yellow solid, which was washed several times with ether to provide 81.2 g (83%) of the title compound m.p. 100-101 °C.

NMR ($CDCl_3$) w 1.08 (t, J=8Hz, $CH_2CH_2CH_3$), 1.86 (M, $CH_2CH_2$, $CH_3$), 2.45 (t, J=6.5 Hz, $CH_2CH_2OH$), 3.14 (t, J=7.5Hz, S-$CH_2CH_2OH$), 3.44 (s, -CO-$CH_2$-$CH_2$-CO), 3.74 (m, $CH_2CH_2OH$), 3.92-3.95 (-4 AR-$OCH_3$), 4.08 (t, J=7.5Hz, $OCH_2CH_2CH_3$), 7.3 (s, trimethoxyphenyl H-2, H-6), 7.50 (d, J=15Hz, thiophenyl, H-6), 7.72 (d, J=1.5Hz, thiophenyl, H-2).

Step C: Preparation of 1-(5-(2-Hydroxyethylsulfonyl-4-propoxy-3-methoxyphenyl)-4-(3,4,5-trimethoxyphenyl)-butan-1-4-dione

3-Chloroperbenzoic acid (69 g, 0.39 mole) was added slowly at 0 °C to a stirred solution of 1-(5-(2-hydroxyethylthio-4-propoxy-3-methoxyphenyl)-4-(3,4,5-trimethoxyphenyl)-butan-1,4-dione (80 g, 0.162 mole) in dichloromethane (800 mL). The temperature was slowly brought to ambient temperature, and the mixture stirred for 3-4 hours. The mixture was then cooled to 0°, filtered to remove 3-chlorobenzoic acid, and the filtrate was evaporated to a small volume. The residue was extracted with ethyl acetate, washed with saturated aqueous $NaHCO_3$, water, and brine, dried over anhydrous $MgSO_4$, and evaporated in vacuo to provide a yellow oil. The oil was crystallized from ether/hexane to afford a pale yellow solid which was washed several times with ether to provide 75 g (88%) of the title compound m.p. 154-155 °C.

NMR ($CDCl_3$): δ 1.03 (t, J=8Hz, $CH_2CH_2CH_3$), 1.90 (m, $CH_2CH_2CH_3$), 3.46 (s, $COCH_2CH_2CO$), 3.68 (m, $SO_2CH_2CH_2OH$), 3.94-3.98 (s, 4 Ar $OCH_3$), 4.00 (m, $SO_2CH_2CH_2OH$), 4.22 (T, J=8Hz, $OCH_2CH_2CH_3$), 7.28 (s, Ar-H H-2, H-6), 7.82 (d, J=1.5Hz, Ar-H H-6), 8.21 (d, J=1.5Hz Ar-H H-2)

Step D: Preparation of 1-(5-(2-Hydroxyphenylsulphonyl-4-propoxy-3-methoxyphenyl)-4-(3,4,5-trimethoxyphenyl)-butan-1,4-diol

Sodium borohydride (7 g, 0.186 mole) was added slowly to a suspension of 1-(5-(2-hydroxyethyl)-sulfonyl-4-propoxy-3-methoxyphenyl)-4-(3,4,5-trimethoxyphenyl)butan-1,4-dione (75 g, 0.142 mole) in a mixture of dry THF and methanol (800 mL, 3:1 v/v) at about 0-5 °C. The mixture was slowly brought to room temperature and stirred for 3-4 hours. After the completion of the reaction, solvent was evaporated, and the residue was taken up in ethyl acetate, washed with 1N HCl, water, and brine, dried over anhydrous $MgSO_4$, and evaporated in vacuo to give title compound as a thick syrup in near quantitative yield. The product was used in the next step without further purification.

Step E: Preparation of trans-2-(5-(2-hydroxyethylsulphonyl)-4-propoxy-3-methoxyphenyl)-5-(3,4,5-trimethoxyphenyl)-tetrahydrofuran

Trifluoroacetic acid (10%, v/v) in chloroform (500 mL, stabilized with ethanol) was added to a stirred 0 °C solution of 1-(3-(2-hydroxyethylsulfonyl)-4-propoxy-5-methoxyphenyl)-4-(3,4,5-trimethoxyphenyl)butan-1,4-diol (75 g, 0.142 mole) in chloroform (500 mL, stabilized with ethanol). The temperature was slowly raised to room temperature and the mixture stirred for 3-4 hours. Anhydrous $Na_2CO_3$ (0 g, 0.75 mole) was added to this solution, and the mixture was stirred vigorously for 30 minutes. The solid was filtered off, and the filtrate was evaporated to a residue, which was purified by flash column chromatography on $SiO_2$ eluted with hexane-ethyl acetate (6:1, v/v) to afford the desired trans isomer which is more mobile than the cis isomer. The product was recrystallized from methylene chloride-hexane to give 34.7 g (48%) of pure trans-2-(5-(2-hydroxyethylsulphonyl)-4-propoxy-3-methoxyphenyl)-5-(3,4,5-trimethoxyphenyl)tetrahydrofuran, m.p. 141-142 °C

NMR ($CDCl_3$) δ: 1.03 (t, J=7.8Hz, $CH_2CH_2CH_3$), 1.85 (m, $CH_2CH_2CH_3$), 2.01, 2.46 (m, H-3 and H-4), 3.64 (m, $SO_2CH_2CH_2OH$) 3.84, 3.86, 3.88 (35, 4 $OCH_3$), 4.89 (m, $SO_2CH_2CH_2OH$), 4.07 (t, J=8Hz, $OCH_2CH_2CH_3$), 5.13 (m, H-2 and H-5) 6.62 (s, Ar-H H-2 and H-6), 7.32 (d, J=2Hz, Ar-H H-6), 7.52 (d, J=2Hz, Ar-H H-2)

EXAMPLE 25

(-)-(2S, 5S)-2-(5-(2-hydroxyethylsulfonyl)-4-propoxy-3-methoxyphenyl)-5-(3,4,5-trimethoxyphenyl)-tetrahydrofuran

Step A: Preparation of 1-(5-)2-t-butyldimethylsilyloxyethylsulfonyl)-4-propoxy-3-methoxypehnyl-4-oxo-4-(3,4,5-trimethoxyphenyl)-1-butanol

A solution of 1-(5-(2-t-butyldimethylsilyloxyethylsulfonyl)-4-propoxy-3-methoxyphenyl)-4-(3,4,5-trimethoxyphenyl)butan-1,4-dione (1.076 g, 1.70 mmole) at 0° C in dry THF (40 mL) under nitrogen was treated with lithium tri-t-butoxyaluminum hydride (500 mg, 2 mmole), and the mixture was stirred at 0° C for 1 hour. The reaction was allowed to warm to room temperature and stirred for 3 hours. The mixture was poured into ethyl acetate (500 mL), and the ethyl acetate solution was washed with 1N HCl (2 x 100 mL), water and brine, dried over anhydrous $MgSO_4$, and evaporated to dryness. The crude product was purified by flash column chromatography on $SiO_2$ and eluted with 40% hexane-ethyl acetate (3:2 v/v) to provide 757 mg (70%) of the pure title product as a thick syrup.
NMR ($CDCl_3$): δ 0.81 (s, $SO_2CH_2CH_2OS_2(CH_3)_3C(CH_3)_3$), 1.08 (t, J=8Hz, $CH_2CH_2CH_3$, 1.92 (m, $CH_2CH_2CH_3$), 2.44 (m, $CH_2CHOH$), 2.88 (d, J-4Hz, $CH_2CHOH$), 3.18 (m, $COCH_2$), 3.9-4.06 (S, 4, $ArOCH_3$ and m, $SO_2CH_2CH_2OSi(CH_3)2C(CH_3)3$) 4.16 (t, J-7.5Hz, $OCH_2CH_2CH_3$), 4.91 (m, $CH_2CHOH$), 7.28 (S, AR-H H-2 and H-6), 7.32 (d, J=1.5Hz, Ar-H H-6), 7.48 (d, J=1.5Hz, Ar-H H-2).

Step B: Preparation of 1-(5-(2-hydroxyethylsulfonyl)-4-propoxy-3-methoxyphenyl)-4-(3,4,5-trimethoxyphenyl)-4-oxo-butan-1-yl-(R)-(O)-methylmandelate

At ambient temperature, a solution of 1-(5-(2-t-butyldimethylsilyloxyethylsulfonyl)-4-propoxy-3-methoxyphenyl)-4-oxo-4-(3,4,5-trimethoxyphenyl)-1-butanol (640 mg, 1 mmole) in dry $CH_2Cl_2$ (20 ml) was treated with EDAC•HCl (380 mg, 2 mmole), R(-)-O-methylphenylacetic acid (200 mg, 1.2 mmole), and a catalytic amount of 4-dimethylaminopyridine, and stirred for 3 hours. The reaction mixture was diluted with ether, washed with water and brine, dried over anhydrous $MgSO_4$, and evaporated to dryness to provide a nearly quantitative yield of 1-(5-(2-t-butyldimethylsilyloxyethylsulfonyl-4-propoxy-3-methoxyphenyl)-4-(3,4,5-trimethoxyphenyl)-4-oxo-butan-1-yl-(R)-(O)-methylmandelate. This crude product was dissolved in THF (20 ml), treated with 1N HCl (6 mL), and stirred at room temperature for 4-6 hours. The mixture was diluted with ether, washed with water and brine, dried over anhydrous $MgSO_4$, and evaporated in vacuo to provide a pale yellow oil. The oil was purified by flash chromatography on $SiO_2$ to afford 650 mg of a mixture of diasteriomeric mandelates. The diastereomeric esters were then carefully separated using flash column chromatography on $SiO_2$ to provide 210 mg (31%) of the more mobile ester 1-(5-(2-hydroxyethylsulfonyl)-4-propoxy-3-methoxyphenyl)-4-(3,4,5-trimethoxyphenyl)-4-oxo-butan-1-yl(R)-(O)-methylmandelate, 212 mg (32%) of the less mobile ester and 98 mg (15%) of a mixture of the two esters (total yield: 78%). Each diastereoisomer was ≥ 95% pure by NMR.
NMR ($CDCl_3$): δ (1.04 (t, J=7.5Hz, $CH_2CH_2CH_3$), 1.86 (m, $CH_2CH_2CH_3$), 2.2 (m, $CH_2CHO$-mandelate), 2.68 (t, J=7Hz, $COCH_2$), 3.38 (s, $PhCHOCH_3$), 3.64 (m, $SO_2CH_2CH_2OH$), 3.8-4.00 (s, 4 $ArOCH_3$, and m, $SO_2CH_2CH_2OH$), 4.12 (t, J=7.0Hz $OCH_2CH_2CH_3$), 4.82 (s, $PHCHOCH_3$), 5.86 (t, J=6.5Hz, $CH_2CHO$-mandelate), 7.02 (s, trimethoxyphenyl H-2, H-6), 7.09 (d, J=1.5Hz, sulfonylphenyl H-6), 7.24-7.48 (m, $PhCHOCH_3$), 7.53 (d, J=1.5Hz, sulfonylphenyl H-2).
The less mobile esters, NMR ($CDCl_3$): 1.02 (t, J=7.5Hz $CH_2CH_2CH_3$), 1.84 (m, $CH_2CH_2CH_3$), 2.29 (m, $CH_2CHO$-mandelate), 2.72 (t, J=7Hz $SO_2CH_2CH_2OH$), 2.96 (t, J=7Hz, $COCH_2$), 3.37 (s, $PhCHOCH_3$), 3.75 (m, $SO_2CH_2CH_2OH$), 3.64 (s, 1 Ar, $OCH_3$), 3.8-4.00 (s, 3-$ArOCH_3$ and m, $SO_2CH_2CH_2OH$), 4.06 (r, J=7Hz, $CH_2CH_2CH_3$), 4.77 (s, $PhCHOCH_3$), 5.9 (t, J=6.5Hz, $CH_2CHO$-mandelate), 6.82 (d, J=1.5Hz, sulfonylphenyl H-6), 7.14 (s, trimethoxyphenyl, H-2, H-6), 4.24-4.38 (m, $PhCHOCH_3$ and d, sulfonylphenyl H-2).

Step C: Preparation of (-)-(2S,5S)-2-(3-(2-hydroxyethylsulfonyl)-4-propoxy-5-methoxyphenyl)-5-(3,4,5-trimethoxyphenyl)tetrahydrofuran

A solution of the more mobile fraction of 1-(5-(2-hydroxyethylsulfonyl)-4-propoxy-3-methoxyphenyl)-4-(3,4,5-trimethoxyphenyl-4-oxo-butan-1-yl(R)-(O)-methylmandelate (200 mg, 0.300 mmole) in dry THF (15 mL) at 0° C under nitrogen atmosphere was treated with lithium aluminum hydride (20 mg, 0.5 mmole), and stirred for 2 hours. After the usual workup, the crude (1S, 4RS)-1-(5-(2-hydroxyethylsulfonyl)-4-propoxy-3-methoxyphenyl)-4-(3,4,5-trimethoxyphenyl)-butan-1,4-diol was dissolved in chloroform (2 mL, stabilized with ethanol), chilled to 0°C, stirred, and treated with 10% (v/v) of trifluoroacetic acid in chloroform (2 mL, stabilized with ethanol). After 4 hours at 0°C, anhydrous $Na_2CO_3$ (1.0 mg, 9.4 mmole) was added, and the mixture was stirred for additional 15 minutes. The mixture was then filtered, and the filtrate was evaporated to a syrup which was purified by preparative TLC $SiO_2$ plates developed with 3:2 (v/v) ethyl acetate-hexane to provide 80 mg (52%) of (-)-(2S,5S)-2-(5-2-hydroxyethylsulfonyl)-4-propoxy-3-methoxyphenyl)-5-(3,4,5-trimethoxyphenyl)tetrahydrofuran, m.p. 111-112°C; $[\alpha]_D$ -61.4° (c=1, $CHCl_3$).

Following substantially the same procedures, there was prepared 75 mg (45%) of (+)- (2R, 5R)-2-(5-(2-hydroxy-ethylsulfonyl)-4-propoxy-3-methoxyphenyl)-5-(3, 4,5-trimethoxyphenyl)tetrahydrofuran, m.p. 114-115°C, $[\alpha]_D$ +60.6 (c=1, $CHCl_3$].

## EXAMPLE 26

### 1-(5-Iodo-4-hydroxy-3-methoxyphenyl)-4-(3,4,5-trimethoxyphenyl)-butan-1,4-dione

#### Step A: Preparation of 5-Iodo-4-trimethylsilyloxy-3-methoxybenzaldehyde

5-Iodovanillin (223.76 g, 0.805 mole) was dissolved in dry THF (1.12 L) and to this solution was added bis-trimethylsilylacetamide (208.8 mL, 0.845 mole). The mixture was stirred for 3-4 hours under nitrogen, then evaporated under vacuum to remove THF and excess silyacetamide to provide the title compound as an orange colored oil in near quantitative yield, which was used without further purification.

#### Step B: Preparation of 1-(5-Iodo-4-hydroxy-3-methoxyphenyl)-4-(3,4,5-trimethoxyphenyl)-butan-1,4-dione

5-Iodo-4-trimethylsilyloy-3-methoxybenzaldehyde from Step A in 570 mL dimethyl formamide was treated with 3,4,5-trimethoxyphenyl vinyl ketone (179 g, 0.805 mole) and 3-ethyl-5-(2-hydroxyethyl)-4-methylthiazolium bromide (61.9 g, 0.245 M). Triethylamine (320 mL) was added, and the resulting mixture was stirred at 70°C under nitrogen atmosphere for 2 hours. The mixture was cooled, filtered, and washed with MeOH (500 mL). The resulting solution was cautiously acidified with 6N HCL to pH 1.5 and a heavy yellow precipitate began to form. This mixture was stirred at room temperature for 2 hours to ensure complete desilyation. The precipitate was filtered, washed with $H_2O$ (4 x 1L), and redissolved in a minimum volume of $CH_2Cl_2$. The solution was washed with brine, dried over anhydrous $MgSO_4$, and evaporated in vacuo to provide a white solid. The solid was washed with MeOH and dried under high vacuum to provide 287.7 g (71%) of the title compound as a white solid (m.p. 189-190°C).

NMR ($CDCl_3$): $\delta$ 3.41 (t, J=4Hz, $COCH_2CH_2CO$), 3.94, 3.96 (s, 4 Ar $OCH_3$), 7.3 (s, trimethoxyphenyl H-2, H-6), 7.52 (d, J=1.5Hz, Ar-H H-6), 8.05 (d, J=1.5Hz, Ar-H H-2).

## EXAMPLE 27

### 1-(5-Iodo-4-alkoxy-3-methoxyphenyl)-4-(3,4,5-trimethoxyphenyl)butan-1,4-dione

Following essentially the same alkylation procedure as described in Example 24, Step A, 1-(5-iodo-4-hydroxy-3-methoxyphenyl)-4-(3,4,5-trimethoxyphenyl)butan-1,4-dione was treated with various alkyl bromides and yielded the alkylated diketones which were further converted to trans-2-(5-iodo-4-alkoxy-4-methoxyphenyl)-5-(3,4,5-trimethoxyphenyl)tetrahydrofurans.

## EXAMPLE 28

Trans-2-(5-(2-hydroxypropylsulfonyl-4-alkoxy-5-methoxyphenyl)-5-(3,4,5-trimethoxyphenyl) tetrahydrofurans

Following essentially the same procedures described in Example 24, but substituting for 2-hydroxyethyl disulfide the reagent 2-hydroxypropyl disulfide, there were prepared the following compounds:

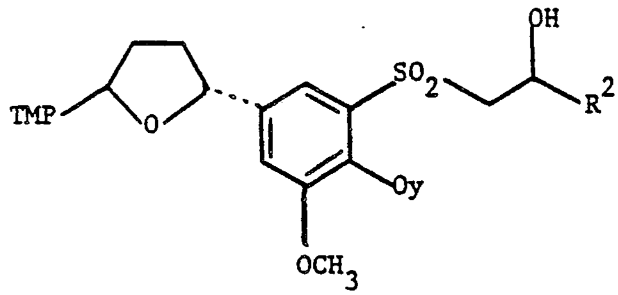

| $R^2$ | Y |
|---|---|
| H | $CH_2CH_2CH_3$ |
| $CH_3$ | $CH_2CH_3$ |
| $(CH_3)_2$ | $CH_2CH_3$ |
| $CH_3$ | $CH_2CH_2CH_3$ |
| $CH_2CH_3$ | $CH_2CH_3$ |
| $CH_2CH_3$ | $CH_2CH_2CH_3$ |
| H | $CH_2(CH_2)_4CH_3$ |
| C=O | $CH_2CH_3$ |
| OEt | |
| C=O | $CH_2CH_3$ |
| C=O | $CH_2CH_3$ |
| $NH_2$ | |

## Claims

1. A compound of formula:

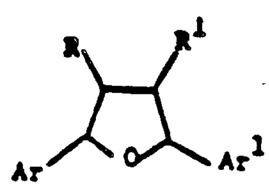

wherein R and $R^1$ independently are

(a) hydrogen;
(b) haloloweralkyl;
(c) halo;
(d) $CONR^2R^3$ wherein $R^2$ and $R^3$ independently represent
1) H;
2) straight or branched chain $C_{1-20}$ alkyl;
3) $C_{3-8}$ cycloalkyl;
4) $C_{2-20}$ alkenyl;
5) $C_{2-20}$ alkynyl;
6) aryl of 6 to 14 carbons;
7) aralkyl;
(e) loweralkenyl;
(f) $-COR^2$;
(g) $-CH_2OR^2$;
(h) loweralkynyl;
(i) $-CH_2NR^2R^3$;
(j) $-CH_2SR^2$;
(k) =O; or
(l) $-OR^2$;
(m) $-R^2$;
Ar and $Ar^1$ are the same or different from each other and are
(a) phenyl or substituted phenyl of formula

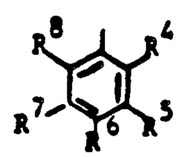

where $R^4$-$R^8$ independently represent
1) $R^2$;
2) YO-; wherein Y is $-R^2$ or $Y^1$ where $Y^1$ represents
(1) $-(CH_2)_{1-6}\ O(CO)R^2$;
(2)

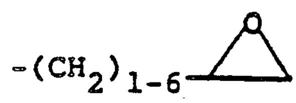 ;

(3) $-(CH_2)_{1-6}-C_{3-8}$ cycloalkyl;
(4) $-(CH_2)_{1-6}-COOR^2$;
(5) $-(CH_2)_{1-6}OR^2$;
(6) $-(CH_2)_{1-6}Ph$;
(7)

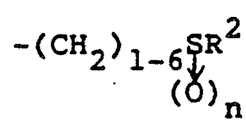

wherein n is 0, 1 or 2;
(8) haloloweralkyl;
(9) $-(CH_2)_{1-7}OSO_2R^2$; or
(10) $(CH_2)_{1-6}NR^2R^3$;
3)

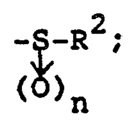

4) $-S(O)_n-(CH_2)_{1-6}OR^2$, where n is 0, 1 or 2;
5) $-OCOCF_3$;
6)

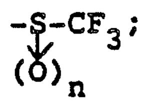

7) $-OCOR^2$;
8) $Y^1$;
9) $R^2R^3N-$;
10)

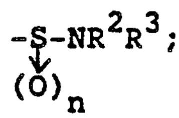

11) $COOR^2$;
12) $-CONR^2R^3$;
13) $-NR^2COR^3$;
14) $-OCONR^2R^3$;
15) $-CRR^9R^9$ wherein $R^8$ is $R^2$ and can be the same as or different from $R^2$;
16) $-(CH_2)_{1-6}OCOR^2$;
17) $-(CH_2)_{1-6}NR^2COR^3$;
18) $-NHCH_2COOR^2$;
19) $-CONR^2COR^3$;
20) $-(CH_2)_{1-6}NR^2R^3$;
21) halo;
22) $-\overset{+}{N}R^2R^3R^9X^-$ wherein $X^-$ is an anion;
23) $-(CH_2)_{1-6}\overset{+}{N}R^2R^3R^9X^-$;
24) $-NR^2SO_2R^3$;
25) $-COR^2$;
26) $-NO_2$;
27) $-CN$;
28) $-(CH_2)_{1-6}CN$;
29) $-N_3$;
30) $-(CH_2)_{1-6}N_3$;
31) $-CONR^2(CH_2)_{1-6}NR^3R^9$;
32) $-PO_3R^2R^3$;
33) $-(CH_2)_{1-6}PO_3R^2R^3$;
34) $-PO_3(R^2)(CH_2)_{1-6}NR^3R^9$;
35) $-R^4-R^5$, $R^5-R^6$, $R^6-R^7$ and $R^7-R^8$ joined together and form a bridge;
36) $SO_2(CH_2)_{1-6}-CO-OR^2$;
37)

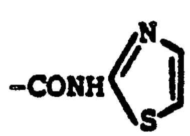 ;

38)

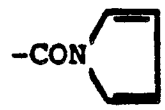 ;

39) $-(CH_2)_{1-6}$ pyridyl;
40) $-SO_2NH(CH_2)_{1-6}$-(2-pyridyl);
41)

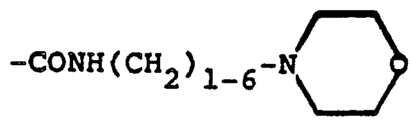 ;

42)

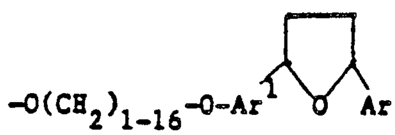 or 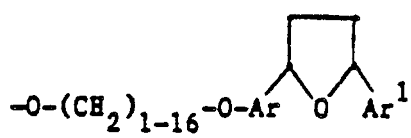 ;

43) heteroaryl as defined below;
44) $-SO_2(CH_2)_{1-6}CONH_2$;
45) $-(CH_2)_{1-6}CONR^2R^3$;
46) $-(CH_2)_{1-6}NR^2SO_2R^3$;
47) $-(CH_2)_{1-6}OSO_2R^2$;
48) $-O(CH_2)_{1-6}-NR^2R^3$;
49) $-CO-NR^2-CO-R^3$;
50) $-S-CO-NR^2R^3$;
51) $-PO_3(CH_2CH_2N(CH_3)_2)(C_2H_5)$;
52) $-S(O)_n(CH_2)_{0-5}CH(OR^2)R^3$;
53) $-S(O)_n(CH_2)_{0-5}CH_2CH(OR^2)-(CO)R^3$;
54) $-S(O)_n(CH_2)_{0-5}CH_2CH(OR^2)-(CO)NHR^3$; or
55) $-S(O)_n(CH_2)_{0-5}CH_2CH(OR^2)-(CO)OR^3$;

(b) monoheteroaryl, di-or polyheteroaryl or fused heteroaryl containing 1 to 3 of any one or more of the heteroatoms N, S or O;

(c) heteroarylalkyl;

(d) heterocycloalkyl; or

(e) heterocycloalkenyl.

2. The compound of Claim 1 which is:

a) trans-2,3-dimethoxy-5-(tetrahydro-5-(3-methoxy-4-propoxy-5-(2-hydroxyethylsulfonyl)-2-furanyl)-pyridine;

b) trans-2-(3,4,5-trimethoxyphenyl)-5-(3-methoxy-4-propoxy-5-(2-hydroxyethylsulfonyl) tetrahydrufuran;

c) trans-2,3-dimethoxy-5-(tetrahydro-5(4-ethoxy-3-methoxy-5-propylsulfonyl)-2-furanyl) pyridine;

d) trans-2-(3,4,5-trimethoxyphenyl)-5-(4-ethoxy-3-methoxy-5-propylsulfonyl)tetrahydrofuran;

e) trans-2-(3,4,5-trimethoxyphenyl)-5-(3-methoxy-4-propoxy-5-N-propylcarboxyamidophenyl) tetrahydrofuran; and

f) trans-2-(3,4,5-trimethoxyphenyl)-5-(3-methoxy-4-propoxy-5-(2-hydroxypropylsulfonyl)phenyl)-tetrahydrofuran.

3. The compound of Claim 1 which is defined as follows:

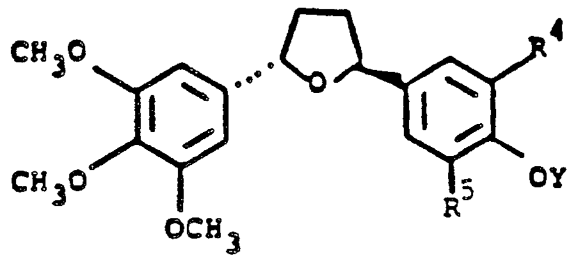

(a) when $R^5$ is $OCH_3$:

| $R^4$ | Y |
|---|---|
| CN | $CH_2CH=CH_2$ |
| I | $CH_2CH=CH_2$ |
| $OCH_3$ | $CH_2$ |
| $OCH_3$ | $CH_2CH=CH_2$ |
| $SCH_3$ | $CH_2CH=CH_2$ |
| $SOCH_3$ | $CH_2CH=CH_2$ |
| $SO_2CH_3$ | $CH_2CH=CH_2$ |
| $NO_2$ | $CH_2CH=CH_2$ |
| $NO_2$ | $CH_2$ 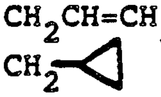 |
| $NH_2$ | $CH_2CH=CH_2$ |
| $NHCH_2CO_2$ | $EtCH_2CH=CH_2$ |
| $N(CH_3)_2$ | $CH_2CH = CH_2$ |
| $^+N(CH_3)_3I^-$ | $CH_2CH=CH_2$ |
| $NHSO_2CH_3$ | $CH_2CH=CH_2$ |
| $N(CH_3)SO_2CH_3$ | $CH_2CH=CH_2$ |
| $NHCOCH_3$ | $CH_2CH_2CH_3$ |
| $CONH_2$ | $CH_2CH_2CH_3$ |
| $CONHCH_2CH_2CH_3$ | $CH_2CH_2CH_3$ |
| CN | $CH_2CH_2CH_3$ |
| $SCH_3$ | $CH_2CH_2CH_3$ |
| $SO_2CH_3$ | $CH_2CH_2CH_3$ |
| $NO_2$ | $CH_3$ 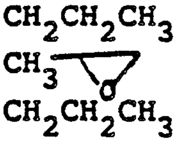 |
| $NO_2$ | $CH_2CH_2CH_3$ |
| $NO_2$ | $CH_2CH_2CH_2CH_3$ |
| $NO_2$ | $CH_2CH_2CH_2CH_2CH_3$ |
| $NO_2$ | $CH_2CO_2CH_2CH_3$ |
| $NH_2$ | $CH_2CH_2CH_3$ |

| | |
|---|---|
| $NHSO_2CH_3$ | $CH_2CH_2CH_3$ |
| $SO_2C_3H_7$ | $(CH_2)_3Cl$ |
| $SO_2C_3H_7$ | $(CH_2)_4Br$ |
| $SO_2(CH_2)_2OC_2H_5$ | $C_3H_7$ |
| $SO_2C_3H_7$ | $CH_2$-(2-pyridyl) |
| $SO_2CH_3$ | $(CH_2)_4\ CH{=}CH_2$ |
| $SO_2(CH_2)_2CH{=}CH_2$ | $C_2H_5$ |
| $SO_2(CH_2)_2C{=}CH$ | $C_2H_5$ |
| $SO_2CH_3$ | $(CH_2)_3$-cyclopentyl |
| $SO_2CH_2C_6H_5$ | $C_2H_5$ |
| $SO_2C_3H_7$ | $(CH_2)_3C_6H_5$ |
| $SO_2CH_2CH_2OH$ | $CH_2CH_2CH_3$ |
| $SO_2CH_2CH(OH)CH_3$ | $C_2H_5$ |

(b) when $R^5$ is other than $OCH_3$:

| $R^4$ | Y | $R^5$ |
|---|---|---|
| $SO_2C_3H_7$ | $C_2H_5$ | $OCONHCH_3$ |
| $SO_2C_3H_7$ | $C_2H_5$ | $OCH_2$-(2-pyridyl) |
| $SO_2C_3H_7$ | $C_2H_5$ | $OCH_2$-(p-F-$C_6H_5$) |

4. The compound of claim 1 having formula

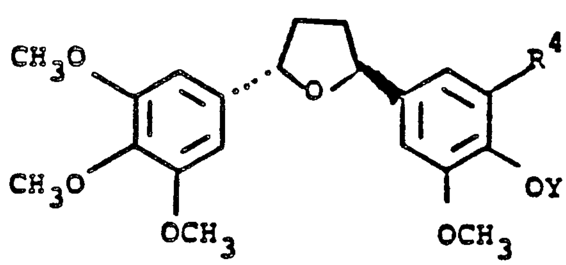

wherein
$R^4$ is (a) $SO_2CH_3$;
(b) $SO_2CH_2CH_2OH$;
(c) $SO_2CH_2CH(OH)CH_3$; and
Y is $C_{1-6}$alkyl.

5. A pharmaceutical composition for treating a disease or a disorder mediated by PAF comprising a pharmaceutical carrier and a therapeutically effective amount of a compound of formula:

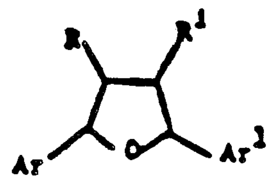

wherein R and $R^1$ independently are

(a) hydrogen;

(b) haloloweralkyl;

(c) halo;

(d) $CONR^2R^3$ wherein $R^2$ and $R^3$ independently represent

1) H;

2) straight or branched chain $C_{1-20}$ alkyl;

3) $C_{3-8}$ cycloalkyl;

4) $C_{2-20}$ alkenyl;

5) $C_{2-20}$ alkynyl;

6) aryl or 6 to 14 carbons;

7) aralkyl;

(e) loweralkenyl;

(f) $-COR^2$;

(g) $-CH_2OR^2$;

(h) loweralkynyl;

(i) $-CH_2NR^2R^3$;

(j) $-CH_2SR^2$;

(k) =O; or

(l) $-OR^2$;

(m) $-R^2$;

Ar and $Ar^1$ are the same or different from each other and are

(a) phenyl or substituted phenyl of formula

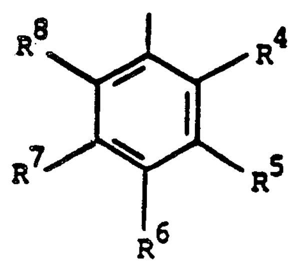

where $R^4$-$R^8$ independently represent

1) $R^2$;

2) YO-; wherein Y is $-R^2$ or $Y^1$ where $Y^1$ represents

(1) $-(CH_2)_{1-6}\ O(CO)R^2$;

(2)

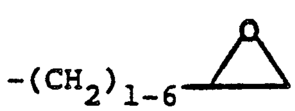 ;

(3) $-(CH_2)_{1-6}-C_{3-8}$ cycloalkyl;

(4) $-(CH_2)_{1-6}-COOR^2$;

(5) $-(CH_2)_{1-6}OR^2$;

(6) $-(CH_2)_{1-6}Ph$;

(7)

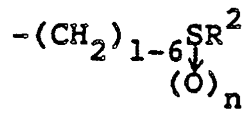

wherein n is 0, 1 or 2;
(8) haloloweralkyl;
(9) $-(CH_2)_{1-7}OSO_2R^2$; or
(10) $(CH_2)_{1-6}NR^2R^3$;
3)

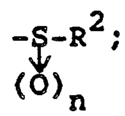

4) $-S(O)_n-(CH_2)_{1-6}OR^2$, where n is 0, 1 or 2;
5) $-OCOCF_3$;
6)

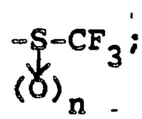

7) $-OCOR^2$;
8) $Y^1$;
9) $R^2R^3N-$;
10)

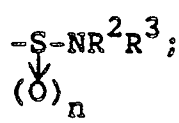

11) $COOR^2$;
12) $-CONR^2R^3$;
13) $-NR^2COR^3$;
14) $-OCONR^2R^3$;
15) $-CR^{23}R^9$ wherein $R^9$ is $R^2$ and can be the same as or different from $R^2$;
16) $-(CH_2)_{1-6}OCOR^2$;
17) $-(CH_2)_{1-6}NR^2COR^3$;
18) $-NHCH_2COOR^2$;
19) $-CONR^2COR^3$;
20) $-(CH_2)_{1-6}NR^2R^3$;
21) halo;
22) $-\overset{+}{N}R^2R^3R^9X^-$ wherein $X^-$ is an anion;
23) $-(CH_2)_{1-6}\overset{+}{N}R^2R^3R^9X^-$;
24) $-NR^2SO_2R^3$;
25) $-COR^2$;
26) $-NO_2$;
27) $-CN$;
28) $-(CH_2)_{1-6}CN$;
29) $-N_3$;

30) $-(CH_2)_{1-6}N_3$;
31) $-CONR^2(CH_2)_{1-6}NR^3R^9$;
32) $-PO_3R^2R^3$;
33) $-(CH_2)_{1-6}PO_3R^2R^3$;
34) $-PO_3(R^2)(CH_2)_{1-6}NR^3R^9$;
35) $-R^4-R^5$, $R^5-R^6$, $R^6-R^7$ and $R^7-R^8$ joined together and form a bridge;
36) $SO_2(CH_2)_{1-6}-CO-OR^2$;
37)

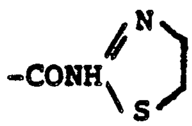 ;

38)

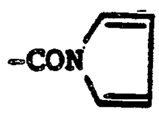 ;

39) $-(CH_2)_{1-6}$ pyridyl;
40) $-SO_2NH(CH_2)_{1-6}$-(2-pyridyl); 41)

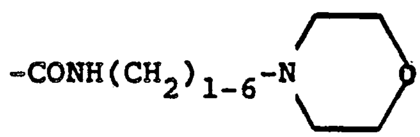 ;

42)

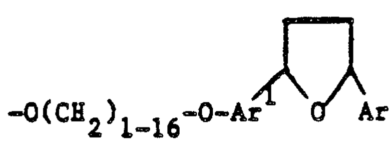 or 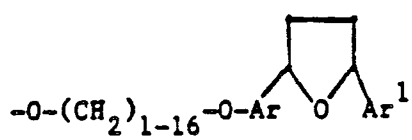 ;

43) heteroaryl as defined below;
44) $-SO_2(CH_2)_{1-6}CONH_2$;
45) $-(CH_2)_{1-6}CONR^2R^3$;
46) $-(CH_2)_{1-6}NR^2SO_2R^3$;
47) $-(CH_2)_{1-6}OSO_2R^2$;
48) $-O(CH_2)_{1-6}-NR^2R^3$;
49) $-CO-NR^2-CO-R^3$;
50) $-S-CO-NR^2R^3$;
51) $-PO_3(CH_2CH_2N(CH_3)_2)(C_2H_5)$;
52) $-S(O)_n(CH_2)_{0-5}CH_2CH(OR^2)R^3$;
53) $-S(O)_n(CH_2)_{0-5}CH_2CH(OR^2)-(CO)R^3$;
54) $-S(O)_n(CH_2)_{0-5}CH_2CH(OR^2)-(CO)NHR^3$; or
55) $-S(O)_n(CH_2)_{0-5}CH_2CH(OR^2)-(CO)OR^3$;

(b) monoheteroaryl, di- or polyheteroaryl or fused heteroaryl containing 1 to 3 of any one or more of the heteroatoms N, S or O;

(c) heteroarylalkyl;

(d) heterocycloalkyl; or

(e) heterocycloalkenyl.

6. The composition of Claim 5 wherein the active compound is:

a) trans-2-3-dimethoxy-5-(tetrahydro-5-(3-methoxy-4-propoxy-5-(2-hydroxyethylsulfonyl)-2-furanyl)-pyridine;

b) trans-2-(3,4,5-trimethoxyphenyl)-5-(3-methoxy-4-propoxy-5-(2-hydroxyethylsulfonyl) tetrahydrofuran;

c) trans-2,3-dimethoxy-5-(tetrahydro-5-(4-ethoxy-3-methoxy-5-propylsulfonyl)-2-furanyl) pyridine;

d) trans-2-(3,4,5-trimethoxyphenyl)-5-(4-ethoxy-3-methoxy-5-propylsulfonyl)tetrahydrofuran;

e) trans-2-(3,4,5-trimethoxyphenyl)-5-(3-methoxy-4-propoxy-5-N-propylcarboxyamidophenyl) tetrahydrofuran; and

f) trans-2-(3,4,5-trimethoxyphenyl)-5-(3-methoxy-4-propoxy-5-(2-hydroxypropylsulfonyl)-phenyl)-tetrahydrofuran.

7. The composition of Claim 5 wherein the active compound is defined as follows:

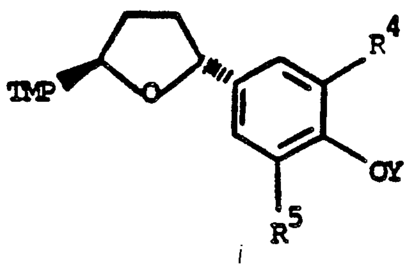

i

(a) when $R^5$ is $OCH_3$:

| $R^4$ | Y |
|---|---|
| CN | $CH_2CH=CH_2$ |
| I | $CH_2CH=CH_2$ |
| $OCH_3$ | $CH_2$ 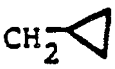 |
| $OCH_3$ | $CH_2CH=CH_2$ |
| $SCH_3$ | $CH_2CH=CH_2$ |
| $SOCH_3$ | $CH_2CH=CH_2$ |
| $SO_2CH_3$ | $CH_2CH=CH_2$ |
| $NO_2$ | $CH_2CH=CH_2$ |
| $NO_2$ | $CH_2$ 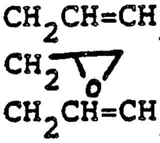 |
| $NH_2$ | $CH_2CH=CH_2$ |
| $NHCH_2CO_2$ | $EtCH_2CH=CH_2$ |
| $N(CH_3)_2$ | $CH_2CH = CH_2$ |
| $^+N(CH_3)_3I^-$ | $CH_2CH=CH_2$ |
| $NHSO_2CH_3$ | $CH_2CH=CH_2$ |
| $N(CH_3)SO_2CH_3$ | $CH_2CH=CH_2$ |
| $NHCOCH_3$ | $CH_2CH_2CH_3$ |
| $CONH_2$ | $CH_2CH_2CH_3$ |
| $CONHCH_2CH_2CH_3$ | $CH_2CH_2CH_3$ |
| CN | $CH_2CH_2CH_3$ |
| $SCH_3$ | $CH_2CH_2CH_3$ |

| | |
|---|---|
| $SO_2CH_3$ | $CH_2CH_2CH_3$ |
| $NO_2$ | $CH_3$ |
| $NO_2$ | $CH_2CH_2CH_3$ |
| $NO_2$ | $CH_2CH_2CH_2CH_3$ |
| $NO_2$ | $CH_2CH_2CH_2CH_2CH_3$ |
| $NO_2$ | $CH_2CO_2CH_2CH_3$ |
| $NH_2$ | $CH_2CH_2CH_3$ |
| $NHSO_2CH_3$ | $CH_2CH_2CH_3$ |
| $SO_2C_3H_7$ | $(CH_2)_3Cl$ |
| $SO_2C_3H_7$ | $(CH_2)_4Br$ |
| $SO_2(CH_2)_2OC_2H_5$ | $C_3H_7$ |
| $SO_2C_3H_7$ | $CH_2$-(2-pyridyl) |
| $SO_2CH_3$ | $(CH_2)_4$ $CH=CH_2$ |
| $SO_2(CH_2)_2CH=CH_2$ | $C_2H_5$ |
| $SO_2(CH_2)_2C≡CH$ | $C_2H_5$ |
| $SO_2CH_3$ | $(CH_2)_3$-cyclopentyl |
| $SO_2CH_2C_6H_5$ | $C_2H_5$ |
| $SO_2C_3H_7$ | $(CH_2)_3C_6H_5$ |
| $SO_2CH_2CH_2OH$ | $CH_2CH_2CH_3$ |
| $SO_2CH_2CH(OH)CH_3$ | $C_2H_5$ |

(b) when $R^5$ is other than $OCH_3$:

| $R^4$ | Y | $R^5$ |
|---|---|---|
| $SO_2C_3H_7$ | $C_2H_5$ | $OCONHCH_3$ |
| $SO_2C_3H_7$ | $C_2H_5$ | $OCH_2$-(2-pyridyl) |
| $SO_2C_3H_7$ | $C_2H_5$ | $OCH_2$-(p-F-$C_6H_5$) |

8. The pharmaceutical composition of claim 7 wherein the compound is of formula

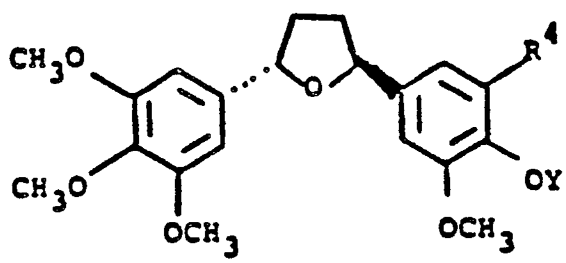

wherein
$R^4$ is (a) $SO_2CH_3$;
(b) $SO_2CH_2CH_2OH$;
(c) $SO_2CH_2CH(OH)CH_3$; and TMP is 3,4,5-trimethoxyphenyl.

9. A process for preparing the compound of formula I according to claim 1.

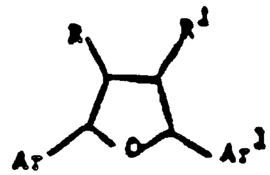

comprising
(a) treating a compound of formula:

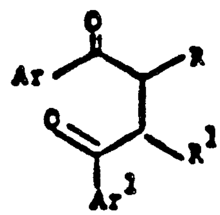

with a reducing agent; or
(b) oxidizing a compound of formula

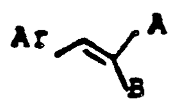

wherein A and B independently are H, hydroxy-$C_{1-6}$alkyl, $C_{1-6}$alkoxy or $C_{1-6}$alkoxycarbonyl, or
(c) treating a compound of formula:

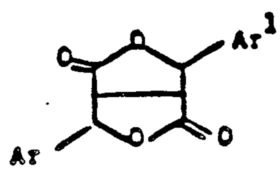

with an aqueous acid.

10. The process of Claim 9 wherein the compound to be prepared is of formula:

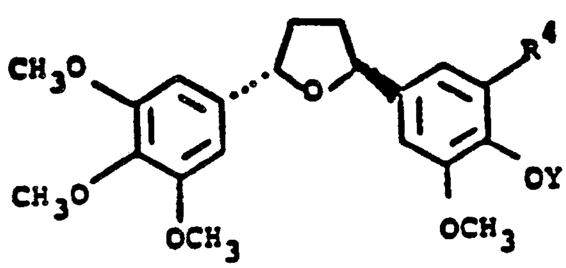

wherein

$R^4$ is (a) $SO_2CH_3$;
(b) $SO_2CH_2CH_2OH$;
(c) $SO_2CH_2CH(OH)CH_3$; and
Y is $C_{1-6}$alkyl.

Claims for the following Contracting States: ES, GR:

1.-A process for preparing new 2,5-diaryl tetrahydrofurans and analogs thereif, having the following formula:

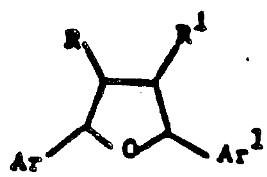

wherein R and $R^1$ independently are
(a) hydrogen;
(b) haloloweralkyl;
(c) halo;
(d) $CONR^2R^3$ wherein $R^2$ and $R^3$ independently represent
1) H;
2) straight or branched chain $C_{1-20}$ alkyl;
3) $C_{3-8}$ cycloalkyl;
4) $C_{2-20}$ alkenyl;
5) $C_{2-20}$ alkynyl;
6) aryl or 6 to 14 carbons;
7) aralkyl;
(e) loweralkenyl;
(f) $-COR^2$;
(g) $-CH_2OR^2$;
(h) loweralkynyl;
(i) $-CH_2NR^2R^3$;
(j) $-CH_2SR^2$;
(k) =O; or
(l) $-OR^2$;
(m) $-R^2$;
Ar and $Ar^1$ are the same or different from each other and are
(a) phenyl or substituted phenyl of formula

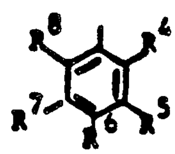

where $R^4$-$R^8$ independently represent
1) $R^2$;
2) YO-; wherein Y is $-R^2$ or $Y^1$ where $Y^1$ represents
(1) $-(CH_2)_{1-6}$ $O(CO)R^2$;
(2)

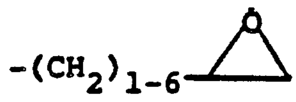 ;

(3) $-(CH_2)_{1-6}-C_{3-8}$ cycloalkyl;
(4) $-(CH_2)_{1-6}-COOR^2$;
(5) $-(CH_2)_{1-6}OR^2$;
(6) $-(CH_2)_{1-6}Ph$;
(7)

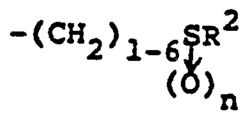

wherein n is 0, 1 or 2;
(8) haloloweralkyl;
(9) $-(CH_2)_{1-7}OSO_2R^2$; or
(10) $(CH_2)_{1-6}NR^2R^3$;
3)

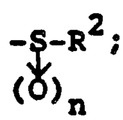

4) $-S(O)_n-(CH_2)_{1-6}OR^2$, where n is 0, 1 or 2;
5) $-OCOCF_3$;
6)

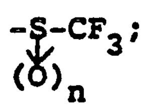

7) $-OCOR^2$;
8) $Y^1$;
9) $R^2R^3N-$;
10)

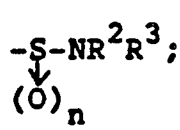

11) $COOR^2$;
37)

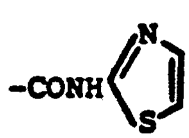 ;

38)

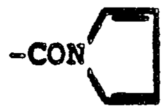 ;

39) $-(CH_2)_{1-6}$ pyridyl;

40) $-SO_2NH(CH_2)_{1-6}$-(2-pyridyl);

41)

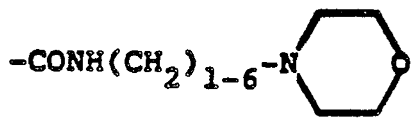 ;

42)

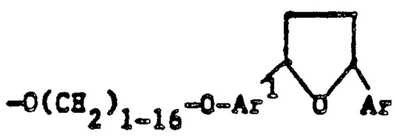 or 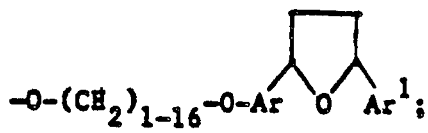

43) heteroaryl as defined below;

44) $-SO_2(CH_2)_{1-6}CONH_2$;

45) $-(CH_2)_{1-6}CONR^2R^3$;

46) $-(CH_2)_{1-6}NR^2SO_2R^3$;

47) $-(CH_2)_{1-6}OSO_2R^2$;

48) $-O(CH_2)_{1-6}-NR^2R^3$;

49) $-CO-NR^2-CO-R^3$;

50) $-S-CO-NR^2R^3$;

51) $-PO_3(CH_2CH_2N(CH_3)_2)(C_2H_5)$;

52) $-S(O)_n(CH_2)_{0-5}CH_2CH(OR^2)R^3$;

53) $-S(O)_n(CH_2)_{0-5}CH_2CH(OR^2)-(CO)R^3$;

54) $-S(O)_n(CH_2)_{0-5}CH_2CH(OR^2)-(CO)NHR^3$; or

55) $-S(O)_n(CH_2)_{0-5}CH_2CH(OR^2)-(CO)OR^3$;

(b) monoheteroaryl, di- or polyheteroaryl or fused heteroaryl containing 1 to 3 of any one or more of the heteroatoms N, S or O;

(c) heteroarylalkyl;

(d) heterocycloalkyl; or

(e) heterocycloalkenyl.

which comprises:

(a) treating a compound of formula

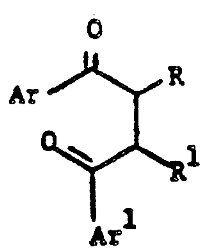

wherein R, $R^1$, Ar and $Ar^1$ are those previously defined, with a reducing agent; or

(b) oxidizing a compound of formula

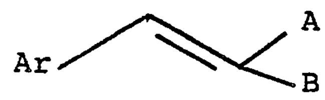

wherein A and B independently are H, hydroxy-$C_{1-6}$ alkyl, $C_{1-6}$ alkoxy or $C_{1-6}$ alcoxycarbonyl; or

(c) treating a compound of formula

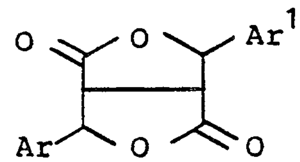

wherein Ar and $Ar^1$ are those previously defined with an aqueous acid.

2.- The process of claim 1, wherein the compound obtained is selected from the group consisting of:

a) trans-2,3-dimethoxy-5-(tetrahydro-5-(3-methoxy-4-propoxy-5-(2-hydroxyethylsulfonyl)-2-furanyl)-pyridine;

b) trans-2-(3,4,5-trimethoxyphenyl)-5-(3-methoxy-4-propoxy-5-(2-hydroxyethylsulfonyl) tetrahydrufuran;

c) trans-2,3-dimethoxy-5-(tetrahydro-5(4-ethoxy-3-methoxy-5-propylsulfonyl)-2-furanyl) pyridine;

d) trans-2-(3,4,5-trimethoxyphenyl)-5-(4-ethoxy-3-methoxy-5-propylsulfonyl)tetrahydrofuran;

e) trans-2-(3,4,5-trimethoxyphenyl)-5-(3-methoxy-4-propoxy-5-N-propylcarboxyamidophenyl) tetrahydrofuran; and

f) trans-2-(3,4,5-trimethoxyphenyl)-5-(3-methoxy-4-propoxy-5-(2-hydroxypropylsulfonyl)phenyl)-tetrahydrofuran.

3.- The process of claim 1, wherein the compound obtained has the following formula

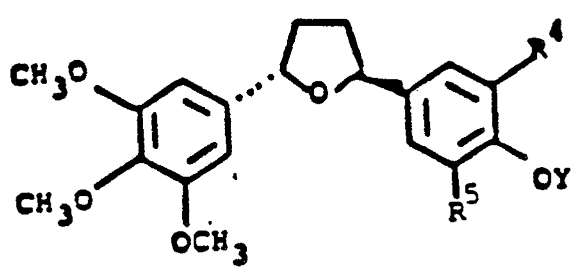

and is defined as follows:

(a) when $R^5$ is $OCH_3$:

| $R^4$ | Y |
|---|---|
| CN | $CH_2CH=CH_2$ |
| I | $CH_2CH=CH_2$ |
| $OCH_3$ | $CH_2$ |
| $OCH_3$ | $CH_2CH=CH_2$ |
| $SCH_3$ | $CH_2CH=CH_2$ |
| $SOCH_3$ | $CH_2CH=CH_2$ |
| $SO_2CH_3$ | $CH_2CH=CH_2$ |
| $NO_2$ | $CH_2CH=CH_2$ |
| $NO_2$ | $CH_2$ 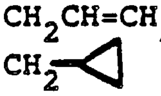 |
| $NH_2$ | $CH_2CH=CH_2$ |
| $NHCH_2CO_2$ | $EtCH_2CH=CH_2$ |
| $N(CH_3)_2$ | $CH_2CH = CH_2$ |
| $^+N(CH_3)_3I^-$ | $CH_2CH=CH_2$ |
| $NHSO_2CH_3$ | $CH_2CH=CH_2$ |
| $N(CH_3)SO_2CH_3$ | $CH_2CH=CH_2$ |
| $NHCOCH_3$ | $CH_2CH_2CH_3$ |
| $CONH_2$ | $CH_2CH_2CH_3$ |
| $CONHCH_2CH_2CH_3$ | $CH_2CH_2CH_3$ |
| CN | $CH_2CH_2CH_3$ |
| $SCH_3$ | $CH_2CH_2CH_3$ |
| $SO_2CH_3$ | $CH_2CH_2CH_3$ |
| $NO_2$ | $CH_3$ 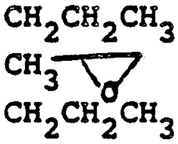 |
| $NO_2$ | $CH_2CH_2CH_3$ |
| $NO_2$ | $CH_2CH_2CH_2CH_3$ |
| $NO_2$ | $CH_2CH_2CH_2CH_2CH_3$ |
| $NO_2$ | $CH_2CO_2CH_2CH_3$ |
| $NH_2$ | $CH_2CH_2CH_3$ |

| | |
|---|---|
| $NHSO_2CH_3$ | $CH_2CH_2CH_3$ |
| $SO_2C_3H_7$ | $(CH_2)_3Cl$ |
| $SO_2C_3H_7$ | $(CH_2)_4Br$ |
| $SO_2(CH_2)_2OC_2H_5$ | $C_3H_7$ |
| $SO_2C_3H_7$ | $CH_2$-(2-pyridyl) |
| $SO_2CH_3$ | $(CH_2)_4\ CH{=}CH_2$ |
| $SO_2(CH_2)_2CH{=}CH_2$ | $C_2H_5$ |
| $SO_2(CH_2)_2C{\equiv}CH$ | $C_2H_5$ |
| $SO_2CH_3$ | $(CH_2)_3$-cyclopentyl |
| $SO_2CH_2C_6H_5$ | $C_2H_5$ |
| $SO_2C_3H_7$ | $(CH_2)_3C_6H_5$ |
| $SO_2CH_2CH_2OH$ | $CH_2CH_2CH_3$ |
| $SO_2CH_2CH(OH)CH_3$ | $C_2H_5$ |

(b) when $R^5$ is other than $OCH_3$:

| $R^4$ | Y | $R^5$ |
|---|---|---|
| $SO_2C_3H_7$ | $C_2H_5$ | $OCONHCH_3$ |
| $SO_2C_3H_7$ | $C_2H_5$ | $OCH_2$-(2-pyridyl) |
| $SO_2C_3H_7$ | $C_2H_5$ | $OCH_2$-(p-F-$C_6H_5$) |

4.- The process of claim 1, wherein the compound obtained has the following formula:

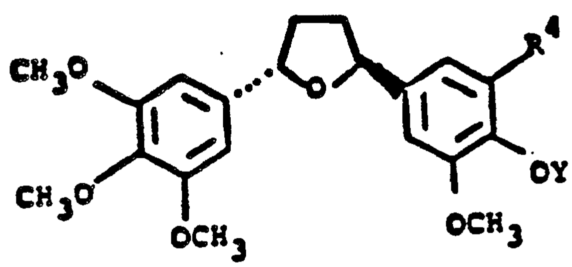

wherein
$R^4$ is (a) $SO_2CH_3$;
(b) $SO_2CH_2CH_2OH$;
(c) $SO_2CH_2CH(OH)CH_3$; and
Y is $C_{1-6}$alkyl.